(19) Europäisches Patentamt / European Patent Office / Office européen des brevets

(11) **EP 4 574 846 A1**

(12) **EUROPEAN PATENT APPLICATION**
published in accordance with Art. 153(4) EPC

(43) Date of publication:
**25.06.2025 Bulletin 2025/26**

(21) Application number: **23864727.5**

(22) Date of filing: **13.09.2023**

(51) International Patent Classification (IPC):
*C07K 19/00* (2006.01)    *C12N 15/62* (2006.01)
*C12N 5/10* (2006.01)    *A61K 39/395* (2006.01)

(52) Cooperative Patent Classification (CPC):
**A61K 39/395; C07K 19/00; C12N 5/10; C12N 15/62**

(86) International application number:
**PCT/CN2023/118594**

(87) International publication number:
**WO 2024/055995 (21.03.2024 Gazette 2024/12)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **14.09.2022 CN 202211116636**

(71) Applicant: **Quaerite Biopharm Research (Beijing)
Co., Ltd.
Beijing 100195 (CN)**

(72) Inventors:
• **HUANG, Jinliang
Beijing 100195 (CN)**
• **HUANG, Wentao
Beijing 100195 (CN)**
• **CHEN, Mengmeng
Beijing 100195 (CN)**

(74) Representative: **HGF
HGF Limited
1 City Walk
Leeds LS11 9DX (GB)**

Remarks:
The complete document including Reference
Table(s) and the Sequence Listing(s) can be
downloaded from the EPO website

(54) **ANTI-VEGFA FUSION PROTEIN, AND PREPARATION METHOD THEREFOR AND USE THEREOF**

(57) Provided herein are an anti-VEGFA fusion protein, and a preparation method thereof and the use thereof. The anti-VEGFA fusion protein has a high affinity with VEGFA, can effectively block the binding of VEGFA to receptor VEGFR, block the VEGFA signaling pathway and effectively block pathological processes such as vascular endothelial cell proliferation and angiogenesis caused by VEGFA, and thus can be potentially applied to the treatment of VEGFA-related diseases.

FIG. 21

EP 4 574 846 A1

**Description**

**TECHNICAL FIELD**

The present disclosure relates to the technical field of biological medicines, and particularly relates to an anti-VEGFA fusion protein, a preparation method thereof, and use thereof.

**BACKGROUND**

Abnormal vascular proliferation is closely associated with the occurrence and development of a variety of diseases, such as various solid tumors and fundus vascular diseases. Studies have shown that vascular endothelial growth factor A (VEGFA) is an important inducer for promoting vascular endothelial cell proliferation and angiogenesis. Therefore, inhibition of the VEGFA signaling pathway may be useful for treating diseases associated with abnormal vascular proliferation, such as wet age-related macular degeneration (AMD), which is the leading cause of blindness in the elderly population. This disease is caused by overexpression of VEGFA in the fundus, leading to choroidal neovascularization and disruption of normal retinal function.

Currently, there are 4 major marketed anti-VEGFA protein drugs for the treatment of wet AMD, including ranibizumab, aflibercept, conbercept, and brolucizumab. Additionally, bevacizumab has also been used off-label for treating wet AMD. The route of administration of these anti-VEGF protein drugs is local injection, such as intravitreal injection, with an administration volume not exceeding 100 μL. The longest administration interval for brolucizumab is 3 months, while the other drugs require injection every 1 to 2 months. Frequent intravitreal injections increase the risk of complications such as elevated intraocular pressure, intraocular inflammation, and retinal detachment, etc. Therefore, there is an urgent clinical need for long-acting anti-VEGFA therapeutic drugs to reduce the number of injections in patients.

Improving the affinity of a drug for a target, i.e., reducing the KD value, can lower the minimum effective concentration of the drug. Among the therapeutic drugs for wet AMD, ranibizumab has a relatively high affinity for VEGFA (KD = 46 pM), while aflibercept and conbercept have a 100-fold higher affinity for VEGFA compared to ranibizumab (KD = 0.5 pM).

The diffusion rate of a drug in the vitreous directly affects the administration interval of the drug. The diffusion rate of a drug is related to its properties such as charge, molecular weight, molecular shape, etc. Drugs with a larger molecular weight diffuse more slowly and have longer half-lives. In a pharmacokinetic study conducted in the vitreous of Dutch belted rabbits, the half-life of ranibizumab (molecular weight: 48 kDa) was 2.81 days, the half-life of bevacizumab (molecular weight: 149 kDa) was 4.22 days, and the half-life of aflibercept (molecular weight: 115 kDa) was 4.58 days.

Therefore, the development of high affinity, long-acting anti-VEGFA fusion proteins is of great significance for improving compliance in patients with wet AMD.

A single domain antibody or a nanobody is a variable region domain of a heavy-chain antibody found naturally in camelids lacking light chains, representing the smallest stable antibody unit with complete antigen-binding functionality. The single domain antibody has a molecular weight of about 13 kDa. It features high thermal stability and good water solubility, and the intrinsic properties of the molecule have great potential for its development into a protein formulation with a higher concentration.

Therefore, the present invention uses VEGFA binding nanobodies as basic active units for the design of bivalent nanobodies, as well as fusion of the nanobodies with Fc fragments to prepare high affinity anti-VEGFA fusion proteins.

**SUMMARY**

In a first aspect of the present disclosure, provided is an anti-VEGFA fusion protein, comprising two or more anti-VEGFA antibodies or antigen-binding fragments thereof, and a Fc fragment, the anti-VEGFA antibody or the antigen-binding fragment thereof comprising CDR-H1, CDR-H2, and CDR-H3 of a heavy chain variable region, wherein

an amino acid sequence of the CDR-H1 comprises SYTMG (SEQ ID NO: 1) or an amino acid sequence having at least 80% identity to SYTMG (SEQ ID NO: 1);
an amino acid sequence of the CDR-H2 comprises $AISKGGYKYX_1X_2VSLEA$ (SEQ ID NO: 2) or an amino acid sequence having at least 80% identity to $AISKGGYKYX_1X_2VSLEA$ (SEQ ID NO: 2);
an amino acid sequence of the CDR-H3 comprises $TRAYGSSRLX_3LAX_4TYEY$ (SEQ ID NO: 3) or an amino acid sequence having at least 80% identity to $TRAYGSSRLX_3LAX_4TYEY$ (SEQ ID NO: 3).

Preferably, the anti-VEGFA fusion protein comprises at least two (e.g., 2, 3, 4, 5, 6, 7, 8, 9, 10, 11, 12, 13, 14, 15, 16, 17, 18, 19, 20, or more) anti-VEGFA antibodies or antigen-binding fragments thereof.

In one specific embodiment of the present disclosure, the amino acid sequences of the CDR-H1, the CDR-H2,

EP 4 574 846 A1

and the CDR-H3 are set forth in SEQ ID NOs: 1, 2, and 3, respectively.

Preferably, the amino acid sequences of the CDR-H1, the CDR-H2, and the CDR-H3 are arranged in the order from the N-terminus to the C-terminus, and the amino acids in the CDRs of the antibody in the present application are defined by using the Kabat numbering system.

Preferably, the linkage between the anti-VEGFA antibody or the antigen-binding fragment thereof and another anti-VEGFA antibody or antigen-binding fragment thereof, and between the anti-VEGFA antibody or the antigen-binding fragment thereof and the Fc fragment, is either direct or indirect.

Preferably, the linkage between other anti-VEGFA antibodies or antigen-binding fragments thereof, between the anti-VEGFA antibody or the antigen-binding fragment thereof and another anti-VEGFA antibody or antigen-binding fragment thereof, between another anti-VEGFA antibody or antigen-binding fragment thereof and the Fc fragment, and between the Fc fragment and the anti-VEGFA antibody or the antigen-binding fragment thereof, is either direct or indirect.

Preferably, the direct or indirect linkage includes a direct or indirect linkage to the N-terminus, C-terminus, and/or internal residues of the anti-VEGFA antibody or the antigen-binding fragment thereof, the other anti-VEGFA antibody or antigen-binding fragment thereof, and/or the Fc fragment.

Preferably, the N-terminus, C-terminus, and/or internal residues of the anti-VEGFA antibody or the antigen-binding fragment thereof are connected to the N-terminus, C-terminus, and/or internal residues of another anti-VEGFA antibody or antigen-binding fragment thereof or the Fc fragment.

In one specific embodiment of the present disclosure, the N-terminus of the anti-VEGFA antibody or the antigen-binding fragment thereof is connected to the N-terminus or C-terminus of another anti-VEGFA antibody or antigen-binding fragment thereof.

In one specific embodiment of the present disclosure, the C-terminus of the anti-VEGFA antibody or the antigen-binding fragment thereof is connected to the N-terminus or C-terminus of another anti-VEGFA antibody or antigen-binding fragment thereof.

In one specific embodiment of the present disclosure, the C-terminus of the anti-VEGFA antibody or the antigen-binding fragment thereof or the C-terminus of another anti-VEGFA antibody or antigen-binding fragment thereof is connected to the N-terminus of the Fc fragment.

Preferably, the indirect linkage is a linkage via a linker.

Preferably, the linker is selected from a linker peptide, an oligopeptide, an oligopeptide polymer, a polypeptide, a polypeptide polymer, PEG, a nucleic acid, a polysaccharide, a fatty chain, biotin, streptavidin, and avidin. Preferably, the linker peptide may be a rigid linker, a flexible linker, or non-essential amino acids; further preferably, the amino acid sequence of the linker peptide comprises one of or a combination of two or more of SEQ ID NOs: 77-91, 152-153, and 156.

Preferably, the two or more anti-VEGFA antibodies or antigen-binding fragments thereof are anti-VEGFA antibodies or antigen-binding fragments thereof with completely identical sequences, anti-VEGFA antibodies or antigen-binding fragments thereof with partially identical sequences, or anti-VEGFA antibodies or antigen-binding fragments thereof with completely different sequences.

Preferably, the anti-VEGFA fusion protein further comprises a signal peptide. The signal peptide is connected at the N-terminus of the anti-VEGFA fusion protein. Further preferably, the amino acid sequence of the signal peptide may be SEQ ID NO: 151 or 155.

In one specific embodiment of the present disclosure, the Fc fragment comprises SEQ ID NO: 134, 135, or 158. In one specific embodiment of the present disclosure, the anti-VEGFA fusion protein comprises, sequentially from the N-terminus to the C-terminus, the first anti-VEGFA antibody or the antigen-binding fragment thereof, the linker peptide, and the second anti-VEGFA antibody or the antigen-binding fragment thereof.

In one specific embodiment of the present disclosure, the anti-VEGFA fusion protein comprises, sequentially from the N-terminus to the C-terminus, the signal peptide, the first anti-VEGFA antibody or the antigen-binding fragment thereof, the linker peptide, and the second anti-VEGFA antibody or the antigen-binding fragment thereof.

In one specific embodiment of the present disclosure, the anti-VEGFA fusion protein comprises, sequentially from the N-terminus to the C-terminus, the signal peptide, the first anti-VEGFA antibody or the antigen-binding fragment thereof, the linker peptide, the second anti-VEGFA antibody or the antigen-binding fragment thereof, and the Fc fragment.

In one specific embodiment of the present disclosure, the anti-VEGFA fusion protein comprises, sequentially from the N-terminus to the C-terminus, the signal peptide, the first anti-VEGFA antibody or the antigen-binding fragment thereof, the linker peptide, the second anti-VEGFA antibody or the antigen-binding fragment thereof, and a tag (e.g., 6×His).

In one specific embodiment of the present disclosure, $X_1X_2$ in SEQ ID NO: 2 represents DS, DA, NT, DT, NA, or NS; $X_3$ in SEQ ID NO: 3 represents R or K, and $X_4$ represents D, N, E, or K.

Preferably, the amino acid sequences of the CDR-H1, the CDR-H2, and the CDR-H3 comprise any one of the following groups (see Table 1):

    A) SEQ ID NOs: 1, 4, and 9;

B) SEQ ID NOs: 1, 5, and 9;
C) SEQ ID NOs: 1, 6, and 10;
D) SEQ ID NOs: 1, 4, and 11;
E) SEQ ID NOs: 1, 7, and 12;
F) SEQ ID NOs: 1, 6, and 11;
G) SEQ ID NOs: 1, 4, and 10;
H) SEQ ID NOs: 1, 5, and 12;
I) SEQ ID NOs: 1, 4, and 12;
J) SEQ ID NOs: 1, 8, and 12; and
K) SEQ ID NOs: 1, 33, and 34.

Table 1. Amino acid sequences of CDR-H1, CDR-H2, and CDR-H3

| Candidate antibody number | SEQ ID NO:x | CDR-H1 | SEQ ID NO:x | CDR-H2 | SEQ ID NO:x | CDR-H3 |
|---|---|---|---|---|---|---|
| V1 | 1 | SYTMG | 4 | AISKGGYKYDSVSLEA | 9 | TRAYGSSRLRLADTYEY |
| V13 | 1 | SYTMG | 5 | AISKGGYKYDAVSLEA | 9 | TRAYGSSRLRLADTYEY |
| V14 | 1 | SYTMG | 6 | AISKGGYKYNTVSLEA | 10 | TRAYGSSRLRLANTYEY |
| V15 | 1 | SYTMG | 4 | AISKGGYKYDSVSLEA | 11 | TRAYGSSRLRLAETYEY |
| V17 | 1 | SYTMG | 7 | AISKGGYKYDTVSLEA | 12 | TRAYGSSRLRLAKTYEY |
| V21 | 1 | SYTMG | 6 | AISKGGYKYNTVSLEA | 11 | TRAYGSSRLRLAETYEY |
| V29 | 1 | SYTMG | 4 | AISKGGYKYDSVSLEA | 10 | TRAYGSSRLRLANTYEY |
| V30 | 1 | SYTMG | 5 | AISKGGYKYDAVSLEA | 12 | TRAYGSSRLRLAKTYEY |
| V31 | 1 | SYTMG | 5 | AISKGGYKYDAVSLEA | 12 | TRAYGSSRLRLAKTYEY |
| V33 | 1 | SYTMG | 4 | AISKGGYKYDSVSLEA | 12 | TRAYGSSRLRLAKTYEY |
| V36 | 1 | SYTMG | 4 | AISKGGYKYDSVSLEA | 9 | TRAYGSSRLRLADTYEY |
| V40 | 1 | SYTMG | 8 | AISKGGYKYNAVSLEA | 12 | TRAYGSSRLRLAKTYEY |
| V41 | 1 | SYTMG | 4 | AISKGGYKYDSVSLEA | 12 | TRAYGSSRLRLAKTYEY |
| V43 | 1 | SYTMG | 4 | AISKGGYKYDSVSLEA | 10 | TRAYGSSRLRLANTYEY |
| V19 | 1 | SYTMG | 33 | AISKGGYKYNSVSLEA | 34 | TRAYGSSRLKLANTYEY |

Preferably, the antibody or the antigen-binding fragment thereof is a nanobody, a chimeric antibody, a Fab fragment, a Fab' fragment, a Fd fragment, a Fd' fragment, a Fv fragment, a dAb fragment, an isolated CDR, a F(ab')$_2$ fragment, a single domain antibody, a single chain antibody molecule, or a linear antibody.

Preferably, the amino acid sequence of the anti-VEGFA antibody or the antigen-binding fragment thereof comprises any one of the amino acid sequences of SEQ ID NOs: 13-32 and 35-61, or has at least 80% identity to any one of the amino acid sequences of SEQ ID NOs: 13-32 and 35-61; the specific sequence numbers are shown in Table 2.

Table 2. Amino acid sequences of antibodies

| SEQ ID NO:x | Antibody number |
|---|---|
| 13 | V1 |
| 14 | V6 |
| 15 | V8 |
| 16 | V13 |
| 17 | V14 |
| 18 | V15 |

(continued)

| SEQ ID NO:x | Antibody number |
|---|---|
| 19 | V17 |
| 20 | V18 |
| 21 | V19 |
| 22 | V21 |
| 23 | V22 |
| 24 | V25 |
| 25 | V29 |
| 26 | V30 |
| 27 | V31 |
| 28 | V33 |
| 29 | V36 |
| 30 | V40 |
| 31 | V41 |
| 32 | V43 |
| 35 | V1-com-78 |
| 36 | V1-com-87 |
| 37 | V1-2m |
| 38 | V1-3m |
| 39 | V1-com-74-78-97 |
| 40 | V1-4m |
| 41 | V1-SA1 |
| 42 | V1-SA3 |
| 43 | V1-DP |
| 44 | V13-2m |
| 45 | V13-4m |
| 46 | V15-2m |
| 47 | V15-4m |
| 48 | V30-2H-78 |
| 49 | V30-2H-87 |
| 50 | V30-2H-2m |
| 51 | V30-3H-2m |
| 52 | V40-3H-2m |
| 53 | V40-3H-4m |
| 54 | V40-4H-2m |
| 55 | V40-4H-4m |
| 56 | V43-1H-78 |
| 57 | V43-1H-87 |
| 58 | V43-1H-2m |
| 59 | V43-3H-78 |

(continued)

| SEQ ID NO:x | Antibody number |
|---|---|
| 60 | V43-3H-87 |
| 61 | V43-4m |

Preferably, the anti-VEGFA fusion protein binds to a human or monkey VEGFA protein, wherein the sequence of the human VEGFA protein is identical to that of the monkey VEGFA protein.

The anti-VEGFA antibody or the antigen-binding fragment thereof may be a humanized antibody or a fully human antibody.

Preferably, the anti-VEGFA antibody or the antigen-binding fragment thereof comprises a humanized sequence, wherein an engineered site of the humanized sequence is located in a non-CDR region, and further preferably, the humanized engineered site is located in a framework region and/or a constant region of the antibody.

In one specific embodiment of the present disclosure, the amino acid sequence of the anti-VEGFA fusion protein comprises any one of the amino acid sequences of SEQ ID NOs: 62-76, 92-133, 136-150, and 159, or has at least 80% identity to any one of the amino acid sequences of SEQ ID NOs: 62-76, 92-133, 136-150, and 159.

In a second aspect of the present disclosure, provided is a chimeric antigen receptor, wherein an extracellular domain of the chimeric antigen receptor comprises the anti-VEGFA fusion protein described above.

Preferably, the chimeric antigen receptor further comprises any transmembrane region and/or intracellular signaling region conventional in the prior art.

In a third aspect of the present disclosure, provided is a nucleic acid encoding the anti-VEGFA fusion protein described above or the chimeric antigen receptor described above.

In a fourth aspect of the present disclosure, provided is a vector comprising the nucleic acid described above. The vector is capable of expression *in vivo* or *in vitro* or *ex vivo.* Preferably, the vector is a prokaryotic expression vector, a viral expression vector, or a eukaryotic expression vector, such as an *Escherichia coli* series vector, a phage, etc.

In a fifth aspect of the present disclosure, provided is a cell comprising the vector described above or the nucleic acid described above.

The cell may be a eukaryotic cell or a prokaryotic cell.

The eukaryotic cell includes animal and plant cells, such as T cells, yeast cells, HEK293 cells, CHO cells, etc. The prokaryotic cell includes, for example, *Escherichia coli,* etc.

In a sixth aspect of the present disclosure, provided is a preparation method for an anti-VEGFA fusion protein, which comprises introducing the nucleic acid described above into a host cell, and inducing expression thereof. In a seventh aspect of the present disclosure, provided is an immune cell expressing the anti-VEGFA fusion protein described above or the chimeric antigen receptor described above.

Preferably, the immune cell includes, but is not limited to, lymphocytes (e.g., T cells, B cells, and NK cells), dendritic cells, monocytes/macrophages, granulocytes, and mast cells.

Preferably, the immune cell is a CAR-immune cell.

In an eighth aspect of the present disclosure, provided is a method for constructing an immune cell, which comprises transfecting an immune cell with a nucleic acid sequence encoding the chimeric antigen receptor described in the present disclosure for expression.

In a ninth aspect of the present disclosure, provided is a product for treating and/or diagnosing a disease, which comprises any one of the following:

A) the anti-VEGFA fusion protein described above;
B) the chimeric antigen receptor described above;
C) the nucleic acid described above;
D) the vector described above;
E) the cell described above; or
F) the immune cell described above.

The product for treating and/or diagnosing a disease targets cells expressing VEGFA, wherein the cells may be cardiomyocytes, renal proximal tubule cells, hepatocytes, vascular endothelial cells, granulosa cells, specialized epithelial cells, mesenchymal cells, macrophages, platelets, dendritic cells, activated T cells, retinal pigment epithelial cells, Muller cells in the retina, astrocytes, osteoblasts, bronchial and alveolar epithelial cells, pericytes, vascular smooth muscle cells, myofibroblasts, keratinocytes, renal mesangial cells, tumor cells, or the like.

Preferably, the product may be a kit or a medicament or a chip or an antibody-drug conjugate, or the like.

The disease is a disease associated with the VEGFA signaling pathway. Further preferably, it may be a tumor, abnormal vascular proliferation, an ophthalmic disease involving angiogenesis (e.g., a fundus vascular disease), or the like.

In a tenth aspect of the present disclosure, provided is an antibody-drug conjugate (ADC), which comprises the anti-VEGFA fusion protein described in the present disclosure that is covalently bound to a drug.

In an eleventh aspect of the present disclosure, provided is a method for detecting VEGFA, which comprises contacting a sample to be tested with the anti-VEGFA fusion protein described above, and subsequently detecting the content of a complex formed by VEGFA and the anti-VEGFA fusion protein.

The detection method is to detect the existence or the content of VEGFA. The existence indicates presence or absence, and the content may be an expression level, a protein concentration, or the like.

In a twelfth aspect of the present disclosure, provided is a method for diagnosing a disease, which comprises taking a sample, contacting the sample with the anti-VEGFA fusion protein, the chimeric antigen receptor described above, the nucleic acid described above, the vector described above, the cell described above, the immune cell described above, or the product for treating and/or diagnosing a disease described above, and detecting the content of a complex formed by VEGFA and the anti-VEGFA fusion protein.

The disease is a disease associated with the VEGFA signaling pathway. Further preferably, it may be a tumor, abnormal vascular proliferation, an ophthalmic disease involving angiogenesis (e.g., a fundus vascular disease), or the like.

In a thirteenth aspect of the present disclosure, provided is use of the anti-VEGFA fusion protein, the chimeric antigen receptor described above, the nucleic acid described above, the vector described above, the cell described above, or the immune cell described above in preparing a product for treating and/or preventing a VEGFA-associated disease, or in preparing a product for blocking VEGFA-mediated vascular endothelial cell proliferation or inhibiting angiogenesis, or in preparing an antibody-drug conjugate or an antibody diagnostic kit or a tracer.

In a fourteenth aspect of the present disclosure, provided is a method for treating and/or preventing a disease, which comprises administering to an individual the anti-VEGFA fusion protein described above, the chimeric antigen receptor described above, the nucleic acid described above, the vector described above, the cell described above, the immune cell described above, or the product for treating and/or diagnosing a disease described above. The disease is a disease associated with the VEGFA signaling pathway. Further preferably, it may be a tumor, abnormal vascular proliferation, an ophthalmic disease involving angiogenesis (e.g., a fundus vascular disease), or the like.

In a fifteenth aspect of the present disclosure, provided is a method for blocking VEGFA-mediated vascular endothelial cell proliferation or inhibiting angiogenesis, which comprises contacting a vascular endothelial cell with the anti-VEGFA fusion protein described above, the chimeric antigen receptor described above, the nucleic acid described above, the vector described above, the cell described above, the immune cell described above, or the product for treating and/or diagnosing a disease described above.

Preferably, the method comprises diluting the anti-VEGFA fusion protein described above, the chimeric antigen receptor described above, the nucleic acid described above, the vector described above, the cell described above, the immune cell described above, or the product for treating and/or diagnosing a disease described above with a medium containing vascular endothelial cells, followed by incubation with an antigen (e.g., VEGFA165). Preferably, the method further comprises the step of resuspending the vascular endothelial cells with the medium after the incubation.

Preferably, the resuspended vascular endothelial cells and the antigen, as well as the anti-VEGFA fusion protein described above, the chimeric antigen receptor described above, the nucleic acid described above, the vector described above, the cell described above, the immune cell described above, or the product for treating and/or diagnosing a disease described above, are cultured on a culture plate.

Preferably, a detection is performed after the culture.

Preferably, the incubation is performed for 0.5-5 h, preferably 1-3 h, e.g., 0.5 h, 1 h, 2 h, 3 h, 4 h, or 5 h.

Preferably, the incubation is performed at a temperature of room temperature to 45 °C, preferably 30-40 °C, e.g., 25 °C, 30 °C, 35 °C, 36 °C, 37 °C, 38 °C, 39 °C, 40 °C, 45 °C, etc.

Preferably, the culture is performed at a temperature of room temperature to 45 °C, preferably 30-40 °C, e.g., 25 °C, 30 °C, 35 °C, 36 °C, 37 °C, 38 °C, 39 °C, 40 °C, 45 °C, etc. Preferably, the culture is performed in an incubator with 5% $CO_2$.

Preferably, the culture is performed for 1-5 days, preferably 2-4 days, e.g., 1 day, 1.5 days, 2 days, 2.5 days, 3 days, 2.5 days, 4 days, 4.5 days, 5 days, etc.

Preferably, the detection is to detect the number of viable vascular endothelial cells.

In a sixteenth aspect of the present disclosure, provided is a method for treating and/or preventing a disease, which comprises contacting the anti-VEGFA fusion protein described above, the chimeric antigen receptor described above, the nucleic acid described above, the vector described above, the cell described above, the immune cell described above, or the product for treating and/or diagnosing a disease described above with a target cell.

The disease is a disease associated with the VEGFA signaling pathway. Further preferably, it may be a tumor, abnormal vascular proliferation, an ophthalmic disease involving angiogenesis (e.g., a fundus vascular disease), or the like.

Preferably, the target cell is selected from cells expressing VEGFA, such as cardiomyocytes, renal proximal tubule cells, hepatocytes, vascular endothelial cells, granulosa cells, specialized epithelial cells, mesenchymal cells, macrophages, platelets, dendritic cells, activated T cells, retinal pigment epithelial cells, Muller cells in the retina, astrocytes, osteoblasts, bronchial and alveolar epithelial cells, pericytes, vascular smooth muscle cells, myofibroblasts, keratinocytes, renal mesangial cells, tumor cells, etc.

In a seventeenth aspect of the present disclosure, provided is use of the anti-VEGFA fusion protein described above, the chimeric antigen receptor described above, the nucleic acid described above, the vector described above, the cell described above, the immune cell described above, or the product for treating and/or diagnosing a disease described above in blocking the VEGFA signaling pathway.

Preferably, the use comprises:

A) use in preparing a medicament for treating and/or preventing a tumor;

B) use in treating and/or preventing a tumor;

C) use in preparing a medicament for treating and/or preventing an ophthalmic disease involving angiogenesis (e.g., a fundus vascular disease), wherein the fundus vascular disease is preferably age-related macular degeneration (AMD), diabetic macular edema (DME), diabetic retinopathy, central retinal vein occlusion, pathological myopia, neovascular glaucoma, etc.;

D) use in treating and/or preventing an ophthalmic disease involving angiogenesis (e.g., a fundus vascular disease), wherein the fundus vascular disease is preferably age-related macular degeneration (AMD), diabetic macular edema (DME), diabetic retinopathy, central retinal vein occlusion, pathological myopia, neovascular glaucoma, etc.; and/or

E) use in inhibiting abnormal vascular proliferation, preferably blocking vascular endothelial cell proliferation. The anti-VEGFA fusion protein described in the present disclosure has a high affinity for VEGFA and is capable of effectively blocking the binding of VEGFA to its receptor VEGFR, blocking the VEGFA signaling pathway, and effectively blocking VEGFA-induced pathological processes such as vascular endothelial cell proliferation, angiogenesis, etc., making it potentially applicable in the treatment of VEGFA-associated diseases.

The "medicament" described in the present disclosure may be used for treating a human or non-human animal, such as a non-human mammal. The medicament may comprise pharmaceutically acceptable carriers, auxiliary materials or salts common in the prior art. The medicament may be administered by any suitable route of administration, such as gastrointestinal routes (e.g., oral administration) or parenteral routes (e.g., intravenous, intramuscular, subcutaneous, intradermal, intraorgan, intranasal, intraocular, instillation, intracerebral, intrathecal, transdermal, intrarectal administrations, etc.). The medicament may be in any suitable dosage form, such as dosage forms for gastrointestinal or parenteral administration. The dosage forms preferably include, but are not limited to, tablets, pills, powders, granules, capsules, lozenges, syrups, liquids, emulsions, microemulsions, suspensions, injections, sprays, aerosols, powder aerosols, lotions, ointments, plasters, pastes, patches, eye drops, nose drops, sublingual tablets, suppositories, aerosols, effervescent tablets, dripping pills, gels, and the like. The various dosage forms of the medicament can be prepared according to conventional production methods in the field of pharmaceuticals. The medicament may comprise the anti-VEGFA fusion protein, the nucleic acid, the vector, the cell, the immune cell, and the like in a weight ratio of 0.01%-99.5% (specifically, 0.01%, 0.1%, 0.5%, 1%, 2%, 3%, 4%, 5%, 6%, 7%, 8%, 9%, 10%, 20%, 30%, 40%, 50%, 60%, 70%, 80%, 90%, 95%, 99%, or 99.5%). The medicament may be prepared as a reagent with a protein concentration of 1-300 mg/mL (e.g., 1 mg/mL, 5 mg/mL, 10 mg/mL, 15 mg/mL, 20 mg/mL, 25 mg/mL, 30 mg/mL, 35 mg/mL, 40 mg/mL, 45 mg/mL, 50 mg/mL, 55 mg/mL, 60 mg/mL, 65 mg/mL, 70 mg/mL, 75 mg/mL, 80 mg/mL, 85 mg/mL, 90 mg/mL, 95 mg/mL, 100 mg/mL, 105 mg/mL, 110 mg/mL, 115 mg/mL, 120 mg/mL, 130 mg/mL, 140 mg/mL, 150 mg/mL, 160 mg/mL, 170 mg/mL, 180 mg/mL, 190 mg/mL, 200 mg/mL, 210 mg/mL, 220 mg/mL, 230 mg/mL, 240 mg/mL, 250 mg/mL, 260 mg/mL, 270 mg/mL, 280 mg/mL, 290 mg/mL, or 300 mg/mL). A single administration dose of the medicament may be 0.1-1000 mg, e.g., 0.1 mg, 0.2 mg, 0.5 mg, 0.75 mg, 1 mg, 1.25 mg, 1.5 mg, 1.75 mg, 2 mg, 2.25 mg, 2.5 mg, 3 mg, 5 mg, 10 mg, 20 mg, 50 mg, 80 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, or 1000 mg.

The "pharmaceutically acceptable" described in the present disclosure refers to the biological activity and properties of neither significantly stimulating an organism nor inhibiting the active substance of the administered product.

The "method for..." described in the present disclosure can be used for the purposes of diagnosing and treating diseases or non-disease conditions.

The "antigen-binding fragment" described in the present disclosure is a portion of an antibody that retains the specific binding activity of the antibody, i.e., any portion of the antibody that is capable of specifically binding to an epitope on a target molecule of the antibody. It includes, for example, Fab, Fv, Fd, Fab', Fab'-SH, $F(ab')_2$, and variants of these fragments, such as the heavy and/or light chain of the antibody, the heavy and/or light chain variable region of the antibody, or a single or 2 or more CDRs from the heavy or light chain of the antibody. Among these, Fab is a monovalent fragment

EP 4 574 846 A1

consisting of VL, VH, CL, and CH1 domains. F(ab')$_2$ is a bivalent fragment comprising two Fab fragments connected by a disulfide bridge at the hinge region. Fd is a Fd fragment consisting of VH and CH1 domains. Fv is a Fv fragment consisting of VL and VH domains of a single arm of an antibody. Fab' is a Fab fragment with one or more cysteine residues at the C-terminus of the CH1 domain. Fab'-SH is a Fab' in which the cysteine residue(s) of the constant domain bear at least one free thiol group. VH represents a heavy chain variable region, VL represents a light chain variable region, CL represents a light chain, and CH1 is CH1 constituting a heavy chain constant region.

The "Fc" region described in the present disclosure comprises two heavy chain fragments containing the CH2 and CH3 domains of the antibody. The two heavy chain fragments form a dimer through two or more disulfide bonds in the hinge region and are held together by the hydrophobic interactions in the CH3 domains.

The "humanized antibody" described in the present disclosure refers to an antibody whose framework regions and/or constant regions (e.g., CH regions) are encoded by human antibody genes or an antibody that is entirely encoded by human antibody genes. In one specific embodiment of the present disclosure, the CDRs of the antibody are not engineered.

The "treating" described in the present disclosure refers to slowing, interrupting, arresting, controlling, stopping, reducing, or reversing the progression or severity of a sign, symptom, disorder, condition, or disease after the disease has begun to progress, but it does not necessarily involve the complete elimination of all disease-related signs, symptoms, conditions, or disorders.

The "preventing" described in the present disclosure refers to the practice of preventing or delaying the onset of a disease or condition or symptom in the body.

The "individual" described in the present disclosure may be a human or a non-human mammal, and the non-human mammal may be a wild animal, a zoo animal, an economic animal, a companion animal, a laboratory animal, or the like. Preferably, the non-human mammal includes, but is not limited to, a pig, a cow, a sheep, a horse, a donkey, a fox, a raccoon dog, a mink, a camel, a dog, a cat, a rabbit, a rodent (e.g., a rat, a mouse, a guinea pig, a hamster, a gerbil, a chinchilla, and a squirrel), a monkey, or the like.

The "comprises" or "comprising" described in the present disclosure is an open-ended expression. When the term is used to describe a sequence of a protein or nucleic acid, the protein or the nucleic acid may consist of the sequence, or may have additional amino acids or nucleotides at one or both ends of the protein or the nucleic acid, while still retaining the same or similar activity as the original sequence.

The "tumor" described in the present disclosure may be any adverse cell proliferation (or any disease that manifests itself as adverse cell proliferation) or neoplasm, or increased predisposition or risk for adverse cell proliferation, neoplasm or a tumor. The tumor may be benign or malignant, and may also be primary or secondary (metastatic). The neoplasm may be any abnormal growth or proliferation of cells and may be located in any tissue. Examples of the tissue include adrenal gland, adrenal medulla, anus, appendix, bladder, blood, bone, bone marrow, brain, mammary gland, cecum, central nervous system (including or excluding brain), cerebellum, cervix, colon, duodenum, endometrium, epithelial cells (e.g., renal epithelial cells), gallbladder, esophagus, glial cells, heart, ileum, jejunum, kidney, lacrimal gland, larynx, liver, lung, lymph, lymph nodes, lymphoblasts, maxilla, mediastinum, mesenterium, myometrium, nasopharynx, omentum, oral cavity, ovary, pancreas, parotid gland, peripheral nervous system, peritoneum, pleura, prostate, salivary glands, sigmoid colon, skin, small intestine, soft tissue, spleen, stomach, testis, thymus, thyroid, tongue, tonsils, trachea, uterus, vulva, and white blood cells. Further preferably, the tumor is selected from prostate cancer, breast cancer, liver cancer, glioma (e.g., neuroglioma), intestinal cancer, cervical cancer, non-small cell lung cancer, lung cancer, pancreatic cancer, gastric cancer, bladder cancer, skin cancer, rhabdomyosarcoma, tongue squamous cell carcinoma, nasopharyngeal cancer, ovarian cancer, placental choriocarcinoma, lymphoma (e.g., non-Hodgkin's lymphoma, Hodgkin's lymphoma, and cutaneous T-cell lymphoma), leukemia, rectal adenocarcinoma, medulloblastoma, meningioma, neurofibroma (e.g., neurofibrosarcoma), ependymoma, schwannoma, astrocytoma, melanoma, mesothelioma, myeloma, chronic granulocytic leukemia, acute myelogenous leukemia, myelodysplastic syndrome, chronic lymphocytic leukemia, epidermoid cancer, colon cancer, thymus cancer, blood cancer, head and neck cancer, and oropharyngeal cancer.

The "homology" described in the present disclosure means that, in the case of using protein sequences or nucleotide sequences, those skilled in the art can adjust the sequences according to the actual work needs, such that the sequences used have (including but not limited to) 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% identity to the sequences obtained with the prior art.

**BRIEF DESCRIPTION OF THE DRAWINGS**

Hereinafter, examples of the present disclosure will be described in detail with reference to the accompanying drawings, in which:

FIG. 1: Coomassie-stained gel images of purified bivalent nanobodies (linker: 9GS).

FIG. 2: ELISA results of bivalent nanobodies (linker: 9GS) binding to VEGFA.

FIG. 3: ELISA results of bivalent nanobodies (linker: 9GS) competing with VEGFR2.

FIG. 4: Comparison of VEGFA binding activity between bivalent and monovalent nanobodies.

FIG. 5: Comparison of activity in competing with VEGFR2 between a bivalent and monovalent nanobody.

FIG. 6: Coomassie-stained gel images of heterodimeric bivalent nanobodies after purification.

FIG. 7: ELISA results of heterodimeric bivalent nanobodies binding to VEGFA.

FIG. 8: ELISA results of heterodimeric bivalent nanobodies competing with VEGFR2.

FIG. 9: Coomassie-stained gel images of purified bivalent nanobodies with different linkers.

FIG. 10: Activity test of tandem bivalent nanobodies with different linkers binding to VEGFA.

FIG. 11: ELISA results of bivalent nanobodies competing with VEGFR2.

FIG. 12: Coomassie-stained gel images of bivalent V1 nanobodies with different linkers after storage at 4 °C for 30 days.

FIG. 13: Absorbance of bivalent V1 nanobodies with different linkers after storage at 4 °C for 30 days.

FIG. 14: Coomassie-stained gel images of bivalent V1 nanobodies with different linkers after storage at 40 °C for 7 days.

FIG. 15: Absorbance of bivalent V1 nanobodies with different linkers after storage at 40 °C for 7 days, followed by storage at room temperature for 23 days.

FIG. 16: Coomassie-stained gel images of purified bivalent humanized nanobodies.

FIG. 17: ELISA results of bivalent humanized nanobodies binding to VEGFA.

FIG. 18: Coomassie-stained gel images of purified monovalent nanobody-Fc fusion proteins.

FIG. 19: ELISA results of monovalent nanobody-Fc fusion proteins or tandem bivalent nanobodies binding to VEGFA.

FIG. 20: Binding and dissociation curves (SPR) of a monovalent nanobody-Fc fusion protein (V1-SA1-Fc-m1) with VEGFA165.

FIG. 21: Coomassie-stained gel images of purified bivalent nanobody-Fc fusion proteins.

FIG. 22: ELISA results of bivalent nanobody-Fc fusion proteins binding to VEGFA.

FIG. 23: Binding and dissociation curves (SPR) of bivalent nanobody-Fc fusion proteins with VEGFA165.

FIG. 24: Binding and dissociation curves (SPR) of a bivalent nanobody-Fc fusion protein and aflibercept with VEGFA165.

FIG. 25: Activity test of a bivalent nanobody-Fc fusion protein 2V1-SA1-3GS-Fc binding to VEGFA from different species.

FIG. 26: Inhibition of VEGFA-mediated HUVEC proliferation by 2V1-SA1-3GS-Fc and aflibercept.

## DETAILED DESCRIPTION

The technical solutions in the examples of the present disclosure will be described clearly and completely below with reference to the drawings. It is apparent that the described examples are only a part of the examples of the present disclosure, but not all of them. Based on the examples of the present disclosure, all other examples obtained by those of ordinary skill in the art without creative work shall fall within the protection scope of the present disclosure.

**Example 1: Preparation and Activity Study of Bivalent Nanobodies**

**1. Bivalentization design of nanobodies**

In order to improve the efficiency of nanobodies in binding to VEGFA and in blocking VEGFA/VEGFR interaction, two nanobodies with identical sequences were linked tandemly to construct a homodimeric bivalent nanobody, followed by a study of its biological activity.

Firstly, a linker 9GS (amino acid sequence: GGGGSGGGS (SEQ ID NO: 78)), which is flexible and changeable in spatial structure and rich in glycine and serine (GS), was selected to connect tandemly two nanobodies with identical sequences. The coding genes of each nanobody were amplified from an expression plasmid of a monovalent nanobody through PCR and constructed into a pVRC8400 expression vector. A signal peptide of MDAMKRGLCCVLLLCGAVFVSPS (SEQ ID NO: 151) was added to the N-terminus of the first nanobody, while a nucleotide sequence encoding the linker 9GS was added to its C-terminus, followed by a nucleotide sequence encoding the second nanobody. In addition, nucleotide sequences encoding the linker GGGGS (SEQ ID NO: 152) and the 6×His tag were added sequentially to the C-terminus of the second nanobody. The two nanobody nucleotide fragments were subjected to homologous recombination with a vector plasmid by using a seamless cloning kit (purchased from Beyotime, Cat. No.: D7010M) to form a correct expression plasmid. Meanwhile, the coding gene sequence of the positive control antibody BI-VEGF ab (the antibody is a nanobody against VEGFA, and the amino acid sequence of the antibody is derived from SEQ ID NO: 57 in the patent US 9527925 B2) was synthesized, and a 6×His tag was added to the C-terminus of the control antibody BI-VEGF ab. The names and numbers of the sequences of the constructed exemplary bivalent nanobodies are shown in Table 3.

Table 3. Names and numbers of amino acid sequences of bivalent nanobodies

| SEQ ID NO:x | Name of nanobody |
|---|---|
| 62 | 2V1-9GS |
| 63 | 2V15-9GS |
| 64 | 2V29-9GS |
| 65 | 2V30-9GS |
| 66 | 2V31-9GS |
| 67 | 2V33-9GS |
| 68 | 2V36-9GS |
| 69 | 2V41-9GS |
| 70 | 2V43-9GS |

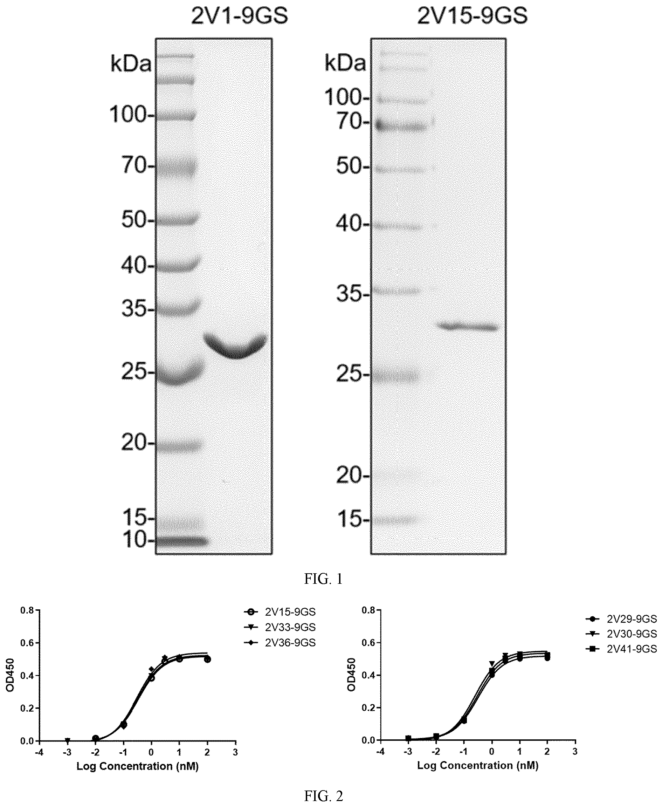

After the expression plasmids for the bivalent nanobodies were confirmed correct by sequencing, endotoxin-free extraction was performed. Polyethylenimine (abbreviated as PEI, linear, MW = 25 kDa, purchased from Polysciences, Cat. No.: 23966-1), a transfection reagent, was used to perform transient transfection on 293F suspension cells. The cell supernatant was collected on day 4 or day 5 after transfection, and Ni-bead affinity purification was carried out. The purified proteins were analyzed by SDS-PAGE, and the molecular weight was in line with the expected value (about 30 kDa). The Coomassie-stained gel images of some of the bivalent nanobodies after purification are shown in FIG. 1.

## 2. Assay for VEGFA binding activity of bivalent nanobodies

The VEGFA binding activity of the purified bivalent nanobodies was detected using the ELISA method.

Antigen human VEGFA165 (label-free, purchased from Sino Biological, Cat. No.: HPLC-10008-HNAH, hereinafter referred to as VEGFA) was diluted with ELISA coating buffer to a final concentration of 0.3 $\mu$g/mL and added to a microplate at 100 $\mu$L/well for coating at 4 °C overnight. After the plate was blocked with 5% skim milk powder, serial dilutions of each bivalent nanobody (0.001 nM, 0.01 nM, 0.1 nM, 1 nM, 3 nM, 10 nM, and 100 nM) were added. The solution used for antibody dilution was PBS containing 0.1% Tween-20 (pH 7.4), i.e., PBST. After being incubated at 37 °C for 1 h, the microplate was washed 3 times with PBST, and a dilution of His-Tag murine monoclonal antibody (purchased from Proteintech, Cat. No.: 66005-1-Ig) was added. The plate was further incubated at 37 °C for 1 h and washed again. Subsequently, a dilution of HRP-labeled goat anti-mouse IgG antibody (HRP-conjugated Affinipure Goat Anti-Mouse IgG (H+L), purchased from Proteintech, Cat. No.: SA00001-1) was added, and the plate was incubated at room temperature for 45 min. After the plate was washed, 100 $\mu$L of TMB (purchased from Tiangen Biotech, Cat. No.: PA107-01) was added to each well for color development at 37 °C for 15 min, followed by the addition of 50 $\mu$L of stopping solution. The absorbance at 450 nm (OD450) was measured using a microplate reader. The signals of some of the bivalent nanobodies binding to VEGFA as a function of antibody concentration are shown in FIG. 2.

The EC50 values of the bivalent nanobodies for binding to VEGFA are shown in Table 4, indicating that the 9 candidate antibodies all had relatively high VEGFA binding activity.

Table 4. EC50 values of bivalent nanobodies for binding to VEGFA

| Bivalent nanobody | EC50 (nM) for binding to VEGFA |
|---|---|
| 2V1-9GS | 0.09 |
| 2V15-9GS | 0.24 |
| 2V29-9GS | 0.31 |
| 2V30-9GS | 0.25 |
| 2V31-9GS | 0.31 |

(continued)

| Bivalent nanobody | EC50 (nM) for binding to VEGFA |
|---|---|
| 2V33-9GS | 0.32 |
| 2V36-9GS | 0.30 |
| 2V41-9GS | 0.27 |
| 2V43-9GS | 0.17 |

**3. Assay for activity of bivalent nanobodies in competing with VEGFR2**

A competitive ELISA was used to further validate the activity of the bivalent nanobodies in blocking the binding of VEGFA to its receptor VEGFR2.

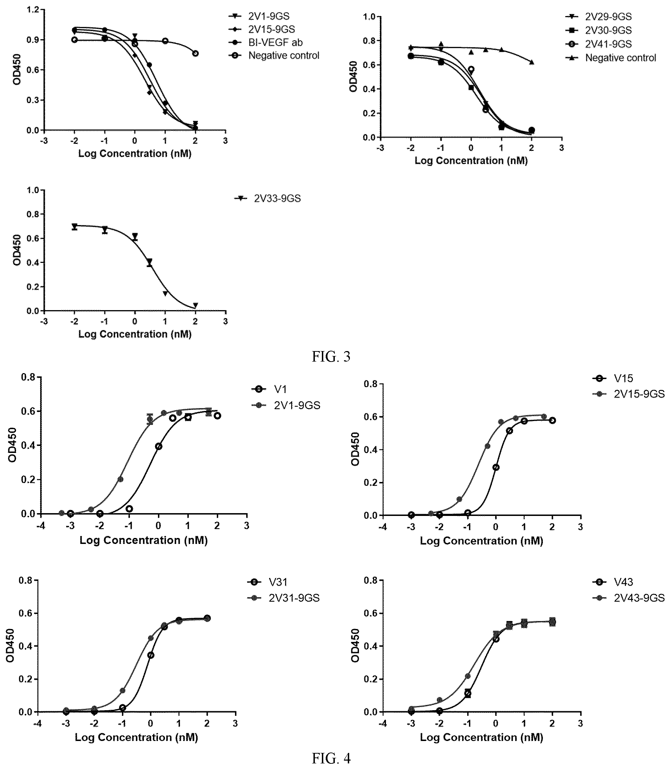

Antigen VEGFA was diluted with ELISA coating buffer (final concentration of 0.53 µg/mL) and added to a 96-well microplate at 100 µL/well for coating at 4 °C overnight. After the plate was blocked with 5% skim milk powder, a VEGFR2 extracellular domain VEGFR2-ECD-Fc (purchased from Sino Biological, Cat. No.: 10012-H02H) at a final concentration of 1 nM was mixed evenly with the serial dilutions of each bivalent nanobody to be tested (0.01 nM, 0.1 nM, 1 nM, 3 nM, 10 nM, and 100 nM), and the resulting mixture was added to the 96-well plate. The plate was incubated at 37 °C for 1 h and washed 3 times with PBST. Subsequently, a dilution of HRP-labeled goat anti-human IgG antibody (HRP-conjugated Goat Anti-Human IgG, purchased from Proteintech, Cat. No.: A21050) was added, and the plate was further incubated at room temperature for 45 min. After the plate was washed, 100 µL of TMB (purchased from Tiangen Biotech, Cat. No.: PA107-01) was added to each well for color development at 37 °C for 15 min, followed by the addition of 50 µL of stopping solution. The absorbance at 450 nm (OD450) was measured using a microplate reader. The competitive ELISA results are shown in FIG. 3.

The comparison of activity of the bivalent nanobodies in competing with VEGFR2 (IC50 value) is shown in Table 5, indicating that the bivalent nanobodies had stronger activity in competing with VEGFR2 as compared to the positive control monovalent nanobody BI-VEGF ab.

Table 5. Activity of bivalent nanobodies in competing with VEGFR2 (IC50 values)

| Bivalent nanobody or control | IC50 (nM) for competing with VEGFR2 |
|---|---|
| 2V1-9GS | 2.97 |
| 2V15-9GS | 2.07 |
| 2V29-9GS | 1.77 |
| 2V30-9GS | 1.41 |
| 2V33-9GS | 3.48 |
| 2V36-9GS | 3.00 |
| 2V41-9GS | 1.97 |
| BI-VEGF ab | 5.20 |

**Example 2: Comparison of VEGFA Binding and Blocking Activity Between Bivalent Nanobodies and Monovalent Nanobodies**

**1.** Comparison of VEGFA binding activity between bivalent nanobodies and monovalent nanobodies

An exemplary ELISA was used to compare the VEGFA binding activity of bivalent nanobodies (2V1-9GS, 2V15-9GS, 2V31-9GS, and 2V43-9GS) with that of their corresponding monovalent nanobodies. The experimental procedure was the same as that described in "2. Assay for VEGFA binding activity of bivalent nanobodies" in Example 1.

The signals of the monovalent and bivalent nanobodies binding to VEGFA as a function of antibody concentration are shown in FIG. 4. The EC50 values of the monovalent and bivalent nanobodies for binding to VEGFA are shown in Table 6, indicating that the VEGFA binding activity of the bivalent nanobodies was stronger than that of the monovalent nanobodies.

Table 6. EC50 values of monovalent and bivalent nanobodies for binding to VEGFA

| Protein | V1 | 2V1-9GS | V15 | 2V15-9GS | V31 | 2V31-9GS | V43 | 2V43-9GS |
|---|---|---|---|---|---|---|---|---|
| EC50 (nM) | 0.52 | 0.09 | 1 | 0.24 | 0.77 | 0.31 | 0.31 | 0.17 |

Furthermore, the binding affinity of the monovalent nanobodies and bivalent nanobodies for VEGFA was exemplarily tested using the surface plasmon resonance (SPR) technology. Using the Biacore 8K instrument (Cytiva), the antigen VEGFA165 (label-free, purchased from Sino Biological, Cat. No.: HPLC-10008-HNAH) as the ligand was coupled onto a CM5 chip through amino-coupling reagents (EDC and NHS), with the coupling level being about 400 RU. The mobile phase buffer was HBS-EP (0.01 M HEPES pH 7.4, 0.15 M NaCl, 3 mM EDTA, 0.005% v/v Tween 20) during the experiment. The analytes were monovalent nanobodies and bivalent nanobodies with the dilution concentrations of 20 nM, 10 nM, 5 nM, 2.5 nM, 1.25 nM, 0.625 nM, and 0.3125 nM. In the kinetic analysis, the binding time between the antigen and the antibody was 240 s, the dissociation time was 1500 s, and the flow rate was 30 $\mu$L/min. In a multi-cycle mode, the chip was regenerated with 100 mM hydrochloric acid. The binding and dissociation curves of the antibodies with VEGFA165 were fitted using the "1:1 binding" model to obtain the binding rate (ka), dissociation rate (kd), and equilibrium dissociation constant (KD), as shown in Table 7.

Table 7. Binding rate (ka), dissociation rate (kd), and equilibrium dissociation constant (KD) of antibodies with VEGFA165

| Ligand | Analyte | ka (1/Ms) | kd (1/s) | KD (M) |
|---|---|---|---|---|
| VEGFA165 | V29 | 2.36E+05 | 1.54E-04 | 6.53E-10 |
| VEGFA165 | V33 | 3.24E+05 | 2.33E-04 | 7.19E-10 |
| VEGFA165 | V43 | 7.43E+05 | 8.63E-05 | 1.16E-10 |
| VEGFA165 | 2V1 | 2.25E+06 | 5.05E-05 | 2.24E-11 |
| VEGFA165 | 2V30 | 4.18E+06 | 9.29E-05 | 2.22E-11 |

From table 7, it can be seen that the bivalent nanobodies had a very high affinity for VEGFA (KD = 22 pM), while the monovalent nanobodies had a relatively high affinity for VEGFA (KD = 116-719 pM), with a difference of 5.3-32.7 times between the two. The results indicate that after the monovalent antibodies were subjected to bivalentization engineering, their binding affinity for the target was significantly improved.

**2.** Comparison of activity in competing with VEGFR2 between bivalent nanobody and monovalent nanobody A competitive ELISA was used to compare the activity of a monovalent nanobody and a bivalent nanobody in blocking VEGFA/VEGFR2 interaction.

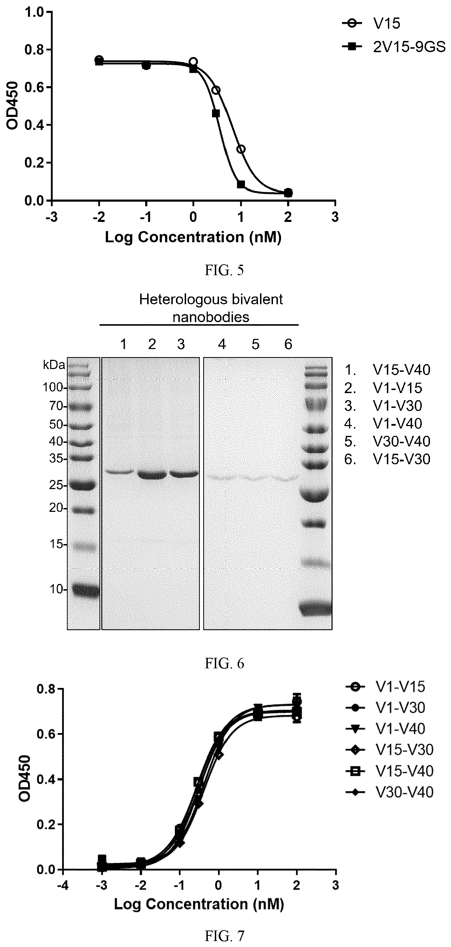

The experimental procedure was the same as that described in "3. Assay for activity of bivalent nanobodies in competing with VEGFR2" in Example 1. The ELISA results for the bivalent nanobody 2V15-9GS and the monovalent nanobody V15 competing with VEGFR2 are shown in FIG. 5.

The activity of the monovalent and bivalent nanobodies in competing with VEGFR2 (IC50 values and maximum inhibition rates) is shown in Table 8.

The formula for calculating the maximum inhibition rate is as follows:

$$(1 - \frac{\text{OD450 at an antibody concentration of 100 nM}}{\text{OD450 at an antibody concentration of 0.01 nM}}) \times 100\%$$

The results show that the activity of the bivalent nanobody in competing with VEGFR2 was stronger than that of the monovalent nanobody.

Table 8: Activity of monovalent and bivalent nanobodies in competing with VEGFR2 (IC50 values and maximum inhibition rates)

| Nanobody | Competing with VEGFR2-IC50 (nM) | Maximum inhibition rate (%) |
|---|---|---|
| V15 | 6.65 | 94.5% |
| 2V15-9GS | 3.61 | 94.6% |

**Example 3: Design and Activity Evaluation of Heterodimeric Bivalent Nanobodies**

**1. Design and preparation of heterodimeric bivalent nanobodies**

(1) Detection of *Tm* values of monovalent antibodies

Eight monovalent antibodies (V1, V15, V29, V30, V31, V36, V40, and V43) were selected, and the buffer in which the antibodies were stored was replaced with: 10 mM His, pH = 6.5, 40 mM NaCl, 5% sucrose, 0.01% Tween-20. The melting temperatures (*Tm*) of the antibodies were measured using the UNCLE instrument (Unchained Labs), and the results are shown in Table 9. The results indicate that these candidate antibodies all possess a certain degree of thermal stability.

Table 9. Melting temperatures (Tm) of monovalent antibodies

| Name of antibody | Tm (°C) |
|---|---|
| V1 | 72.6 |
| V15 | 68.7 |
| V29 | 65.3 |
| V30 | 72.8 |
| V31 | 65.6 |
| V36 | 64.6 |
| V40 | 74.3 |
| V43 | 62.4 |

(2) Design and preparation of heterodimeric bivalent nanobodies

Monovalent nanobodies with different amino acid sequences were connected tandemly by the linker 9GS to construct heterodimeric bivalent nanobodies. The nanobodies V1, V15, V30, and V40 with higher melting temperatures (Tm) were selected to construct heterodimeric bivalent nanobodies. A total of 6 heterodimeric bivalent nanobodies were expressed, with their names, sequence names, and numbers shown in Table 10.

Table 10. Names and numbers of amino acid sequences of heterodimeric bivalent nanobodies

| SEQ ID NO:x | Name of heterodimeric bivalent nanobody |
|---|---|
| 71 | V1-V15 |
| 72 | V1-V30 |
| 73 | V1-V40 |
| 74 | V15-V30 |
| 75 | V15-V40 |
| 76 | V30-V40 |

For the 6 heterodimeric bivalent nanobodies, the plasmid construction, expression and purification methods were the same as those described in "1. Bivalentization design of nanobodies" in Example 1. The Coomassie-stained gel images of the antibodies after Ni-bead affinity purification are shown in FIG. 6. The molecular weight of the target protein was about 30 kDa.

**2. Activity study of heterodimeric bivalent nanobodies**

The purified heterodimeric bivalent nanobodies were evaluated for their VEGFA binding activity, and the experimental procedure was the same as that described in "2. Assay for VEGFA binding activity of bivalent nanobodies" in Example 1. The ELISA results for the heterodimeric bivalent nanobodies binding to VEGFA are shown in FIG. 7.
The EC50 values of the heterodimeric bivalent nanobodies for binding to VEGFA are shown in Table 11. The results show that the 6 heterodimeric bivalent nanobodies had similar VEGFA binding activity (EC50: 0.3-0.4 nM), which was comparable to the activity of the homodimeric bivalent nanobody (constructed by tandemly linking two nanobodies with identical sequences) (EC50: about 0.24 nM, see Table 4 in Example 1).

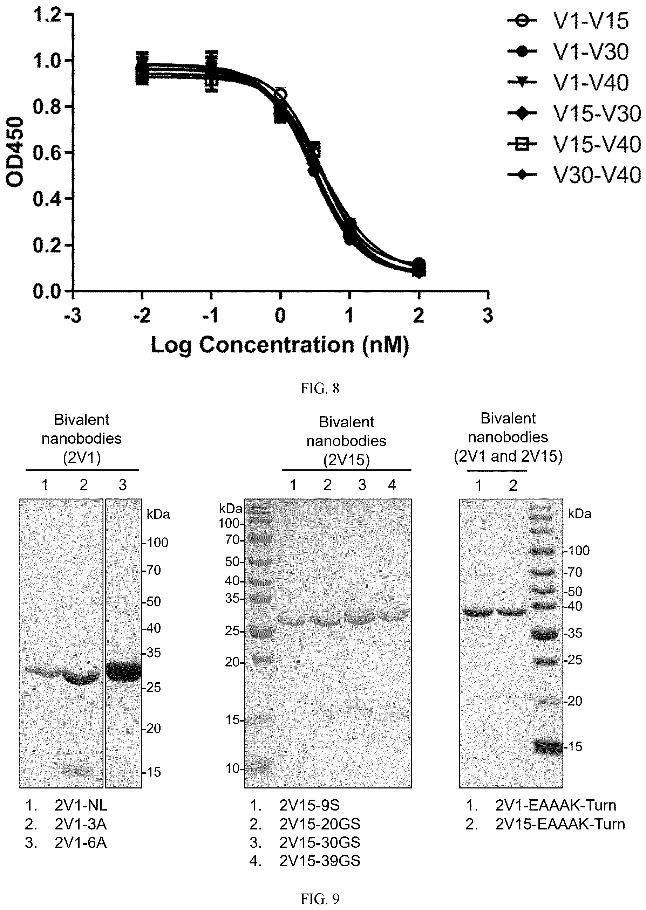

The ELISA results for the heterodimeric bivalent nanobodies competing with VEGFR2 are shown in FIG. 8. The IC50 and inhibition rate results of the competitive ELISA are shown in Table 11. The results show that the heterodimeric bivalent nanobodies had strong activity in competing with VEGFR2 for binding to VEGFA.

Table 11. ELISA results for heterodimeric bivalent nanobodies competing with VEGFR2

| Heterodimeric bivalent nanobody | EC50 (nM) for binding to VEGFA | Competing with VEGFR2 | |
|---|---|---|---|
| | | IC50 (nM) | Maximum inhibition rate (%) |
| V1-V15 | 0.29 | 3.80 | 87.8 |
| V1-V30 | 0.31 | 2.84 | 91.1 |
| V1-V40 | 0.42 | 3.74 | 88.5 |
| V15-V30 | 0.41 | 3.22 | 91.2 |
| V15-V40 | 0.26 | 4.06 | 90.1 |
| V30-V40 | 0.31 | 3.32 | 89.8 |

**Example 4: Effect of Different Linkers on Activity of Bivalent Nanobodies**

In the above examples, the linkers connecting two nanobodies were primarily flexible 9GS linkers, allowing the spatial orientations of the two nanobodies to remain relatively independent without interfering with each other. However, linkers with different properties (e.g., different lengths, hydrophilicity, hydrophobicity, secondary structures, etc.) may affect the spatial configuration and antigen binding efficiency of the antibodies. Therefore, different linkers were prepared and evaluated for their effect on the activity and stability of the bivalent nanobodies.

**1. Design of linkers between nanobodies and preparation of bivalent nanobodies with different linkers**

To understand the effect of linker properties on the biological activity and physicochemical stability of bivalent nanobodies, 16 linkers were designed, varying in properties including linker length, potential to form secondary structures, hydrophilicity, etc. The amino acid sequences of the linkers are shown in Table 12.

Table 12. Amino acid sequences of linkers

| Name of linker | Amino acid sequence of linker | SEQ ID NO:x |
|---|---|---|
| 3GS | GGS | 77 |
| 4GS | GGGGS | 152 |
| 9GS | GGGGSGGGS | 78 |
| 9S | $(S)_9$ | 79 |
| 20S | $(S)_{20}$ | 80 |
| 20GS | GGGGSGGGSGGGSGGGSGGS | 81 |
| 25GS | GGGGSGGGSGGGSGGGSGGGSGGGS | 82 |
| 30GS | GGGGSGGGSGGGGSGGGSGGGSGGGSGGGS | 83 |
| 39GS | GGGGSGGGSGGGGSGGGGSGGGGSGGGGSGGGGSGGGGS | 84 |
| (WL)2* | $(GSTSGSGKPGSGEGSTKG)_2S$ | 85 |
| (AP)5 | $PDS(AP)_5DGSS$ | 86 |
| (AP)10 | $PDS(AP)_{10}DGSS$ | 87 |
| EAAAK-Rigid | $LA(EAAAK)_5AAA$ | 88 |
| EAAAK-Turn | $PDS(EAAAK)_5PDGSS$ | 89 |

(continued)

| Name of linker | Amino acid sequence of linker | SEQ ID NO:x |
|---|---|---|
| 3A | AAA | 90 |
| 6A | AAAAAA | 91 |
| NL | No linker | - |
| *WL represents the Whitlow linker. | | |

Using the nanobodies V1 and V15 as model antibodies, bivalent nanobodies with different linkers were constructed. Their expression vector construction, transient transfection, and purification methods were the same as those described in "1. Bivalentization design of nanobodies" in Example 1. The names and numbers of the sequences of the bivalent nanobodies with different linkers are shown in Table 13.

Table 13. Names and numbers of amino acid sequences of bivalent nanobodies

| SEQ ID NO:x | Name of bivalent nanobody |
|---|---|
| 92 | 2V1-NL |
| 93 | 2V1-3A |
| 94 | 2V1-6A |
| 95 | 2V1-3GS |
| 96 | 2V1-30GS |
| 97 | 2V1-39GS |
| 98 | 2V1-(AP)5 |
| 99 | 2V1-(AP)10 |
| 100 | 2V1-EAAAK-Rigid |
| 101 | 2V1-EAAAK-Turn |
| 102 | 2V15-9S |
| 103 | 2V15-20S |
| 104 | 2V15-20GS |
| 105 | 2V15-25GS |
| 106 | 2V15-30GS |
| 107 | 2V15-39GS |
| 108 | 2V 15-EAAAK-Turn |
| 159 | 2V1-(WL)2 |

The Coomassie-stained gel images of some of the antibodies after Ni-bead affinity purification are shown in FIG. 9. The molecular weight of the target protein was about 30 kDa.

**2. Activity study of tandem bivalent nanobodies with different linkers**

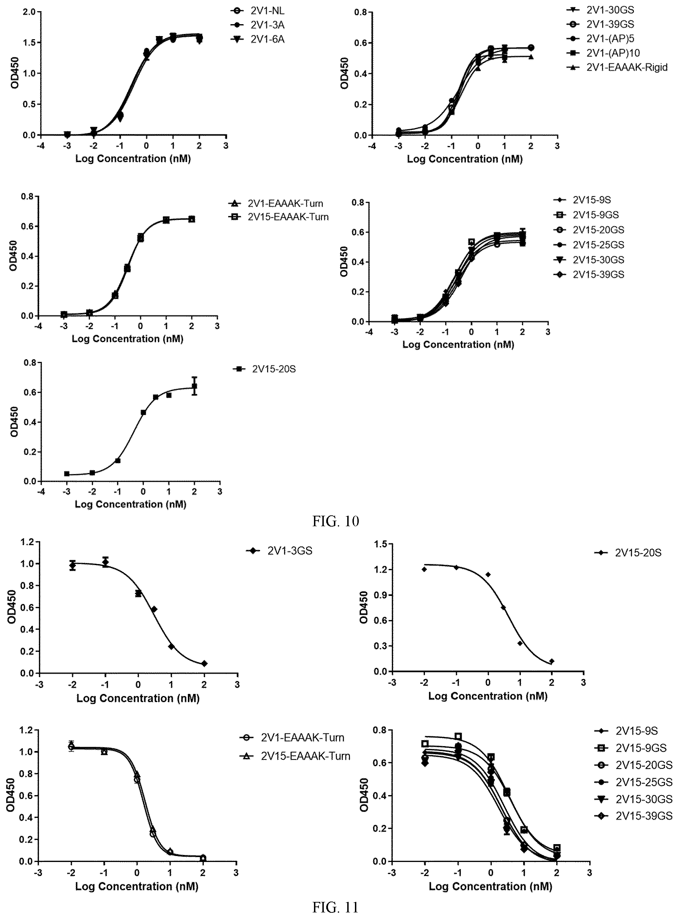

The purified bivalent nanobodies were evaluated for their VEGFA binding activity, and the experimental procedure was the same as that described in "2. Assay for VEGFA binding activity of bivalent nanobodies" in Example 1. The ELISA results for some of the bivalent nanobodies binding to VEGFA are shown in FIG. 10, and the EC50 values for binding to VEGFA are shown in Table 14.

For the bivalent nanobody V1, the effect of different linkers on the EC50 of bivalent V1 was relatively small (EC50: 0.2-0.3 nM), and the activity of bivalent V1 without a linker (2V1-NL) was comparable to that of bivalent V1 with a linker. The activities of tandem bivalent V15 constructed with different linkers were not much different.

Table 14. EC50 values of bivalent nanobodies for binding to VEGFA

| Bivalent nanobody | EC50 (nM) for binding to VEGFA |
|---|---|
| 2V1-NL | 0.29 |
| 2V1-3A | 0.25 |
| 2V1-6A | 0.33 |
| 2V1-30GS | 0.22 |
| 2V1-39GS | 0.18 |
| 2V1-(AP)5 | 0.19 |
| 2V1-(AP)10 | 0.18 |
| 2V1-EAAAK-Rigid | 0.22 |
| 2V 1-EAAAK-Turn | 0.31 |
| 2V15-9S | 0.21 |
| 2V15-20S | 0.43 |
| 2V15-9GS | 0.22 |
| 2V15-20GS | 0.28 |
| 2V15-25GS | 0.24 |
| 2V15-30GS | 0.27 |
| 2V15-39GS | 0.38 |
| 2V 15-EAAAK-Turn | 0.33 |

The ELISA results for the bivalent nanobodies competing with VEGFR2 are shown in FIG. 11. The IC50 and inhibition rate results of the competitive ELISA are shown in Table 15. The results show that for V15, the competitive activity of tandem bivalent V15 constructed with the linker 20S (2V15-20S) was slightly inferior to that of tandem bivalent V15 constructed with other linkers.

Table 15. IC50 and inhibition rate results of ELISA for bivalent nanobodies in competing with VEGFR2

| Bivalent antibody | Competing with VEGFR2-IC50 (nM) | Maximum inhibition rate (%) |
|---|---|---|
| 2V1-3GS | 3.00 | 91.0 |
| 2V 1-EAAAK-Turn | 1.56 | 96.8 |
| 2V15-9S | 3.53 | 88.8 |
| 2V15-20S | 4.38 | 89.8 |
| 2V15-9GS | 3.13 | 88.4 |
| 2V15-20GS | 2.03 | 94.2 |
| 2V15-25GS | 1.66 | 94.5 |
| 2V15-30GS | 1.6 | 94.1 |
| 2V15-39GS | 1.74 | 94.5 |
| 2V 15-EAAAK-Turn | 1.76 | 96.7 |

## Example 5: Effect of Different Linkers on Stability of Bivalent Nanobodies

### 1. Effect of different linkers on stability of bivalent nanobodies at 4 °C

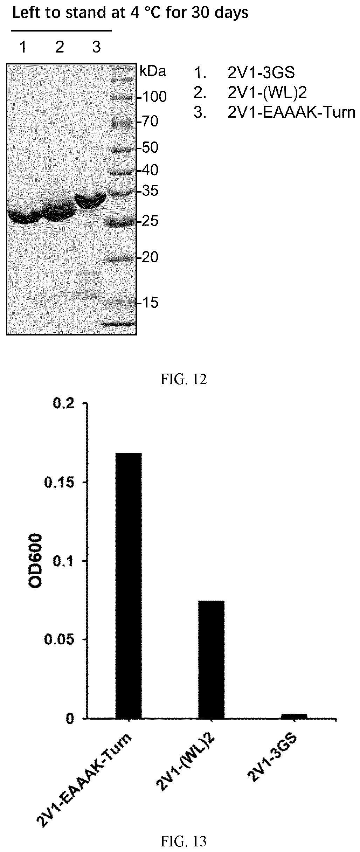

The buffer for bivalent V1 (2V1-3GS, 2V1-(WL)2, and 2V1-EAAAK-Turn) was replaced with: 10 mM His, pH=6.5, 40 mM NaCl, 5% sucrose, 0.01% Tween-20. The protein concentrations were uniformly adjusted to 0.5 mg/mL. At time 0, the protein solutions were clear. After being left to stand at 4 °C for 30 days, the protein solutions were subjected to SDS-PAGE and absorbance detection. The Coomassie-stained gel images of the proteins are shown in FIG. 12. The results show that the bivalent antibody 2V1-EAAAK-Turn exhibited degradation bands with molecular weights lower than the main band. In contrast, no significant degradation fragments were observed in the lanes of 2V1-3GS and 2V1-(WL)2, indicating better chemical stability.

In addition, the absorbance of the samples at a wavelength of 600 nm (OD600) was measured, and the comparison is shown in FIG. 13. It can be seen that the absorbance value of 2V1-3GS was the lowest, and its solution was clearest, suggesting that the degree of aggregation of 2V1-3GS protein was relatively lower compared to the other proteins. Therefore, among the 3 linkers, 3GS was more capable of maintaining the physical stability and chemical stability of bivalent V1.

**2. Effect of different linkers on stability of bivalent nanobodies at high temperature**

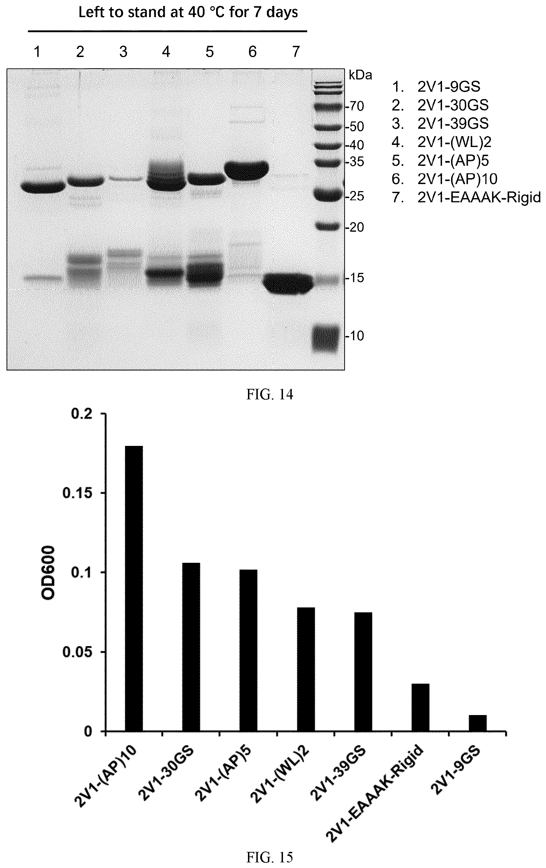

The buffer for bivalent V1 (2V1-9GS, 2V1-30GS, 2V1-39GS, 2V1-(WL)2, 2V1-(AP)5, 2V1-(AP)10, and 2V1-EAAAK-Rigid) was replaced with: 10 mM His, pH=6.5, 40 mM NaCl, 5% sucrose, 0.01% Tween-20. The protein concentrations were uniformly adjusted to 0.5 mg/mL. At time 0, the protein solutions were clear. After being left to stand at 40 °C for 7 days, the protein solutions were subjected to SDS-PAGE. The Coomassie-stained gel images of the proteins are shown in FIG. 14. The results show that the bivalent antibody 2V1-EAAAK-Turn exhibited degradation bands with molecular weights lower than the main band. In contrast, no significant degradation fragments were observed in the lanes of 2V1-3GS and 2V1-(WL)2, indicating better chemical stability.

The results show that among the 7 bivalent V1 proteins, only 2V1-9GS and 2V1-(AP)10 were able to maintain most of the protein without degradation under the acceleration condition of 40 °C.

After being left to stand at 40 °C for 7 days, the samples were further left to stand at room temperature for 23 days. Subsequently, the absorbance of the samples at a wavelength of 600 nm (OD600) was measured, and the comparison is shown in FIG. 15. It can be seen that the absorbance value of 2V1-9GS was the lowest, and its solution was clearest, suggesting that the degree of aggregation of 2V1-9GS protein was relatively lower. Therefore, among the 7 linkers, bivalent V1 containing 9GS had better physical stability and chemical stability. By comparing the chemical stability and physical stability of the bivalent nanobodies containing different linkers at 4 °C and 40 °C, it can be seen that the bivalent nanobodies containing the 3GS and 9GS linkers had better stability performance.

**Example 6: Activity Study of Bivalent Humanized Nanobodies**

**1. Bivalentization design of humanized nanobodies**

In the previous examples, bivalentization design and linker comparison were performed on the original antibodies that had not undergone humanization engineering. Next, bivalent humanized nanobodies were constructed with the 3GS and 9GS linkers, or without a linker (NL). The antibody humanization was conducted by first comparing the candidate antibody sequence with human antibody sequences for homology (through IGBLAST and IMGT databases) and subsequently grafting the CDRs of the candidate antibody into the framework regions of a human antibody with high homology to form a complete humanized antibody. To maintain the activity of the antibody, individual amino acids in the human antibody framework regions needed to be reverted to the sequences at the corresponding positions in the candidate antibody. The humanized antibodies V1, V13, V15, V30, V40 and V43 were used as elements to construct bivalent humanized nanobodies with different linkers. The names and numbers of the amino acid sequences of the constructed bivalent humanized nanobodies are shown in Table 16.

Table 16. Names and numbers of amino acid sequences of bivalent humanized nanobodies

| SEQ ID NO:x | Name of bivalent humanized nanobody |
|---|---|
| 109 | 2V1-SA1-NL |
| 110 | 2V1-SA1-3GS |
| 111 | 2V1-DP-NL |
| 112 | 2V1-DP-3GS |
| 113 | 2V1-DP-9GS |
| 114 | 2V1-2m-3GS |
| 115 | 2V1-3m-3GS |
| 116 | 2V1-SA3-3GS |

(continued)

| SEQ ID NO:x | Name of bivalent humanized nanobody |
|---|---|
| 117 | 2V13-4m-NL |
| 118 | 2V13-4m-3GS |
| 119 | 2V15-4m-NL |
| 120 | 2V15-4m-3GS |
| 121 | 2V30-2H-2m-NL |
| 122 | 2V30-2H-2m-3GS |
| 123 | 2V30-3H-2m-NL |
| 124 | 2V30-3H-2m-3GS |
| 125 | 2V30-3H-2m-9GS |
| 126 | 2V40-3H-2m-NL |
| 127 | 2V40-3H-2m-3GS |
| 128 | 2V40-3H-2m-9GS |
| 129 | V40-3H-4m-NL |
| 130 | V40-3H-4m-3GS |
| 131 | V40-3H-4m-9GS |
| 132 | 2V43-1H-2m-NL |
| 133 | 2V43-1H-2m-3GS |

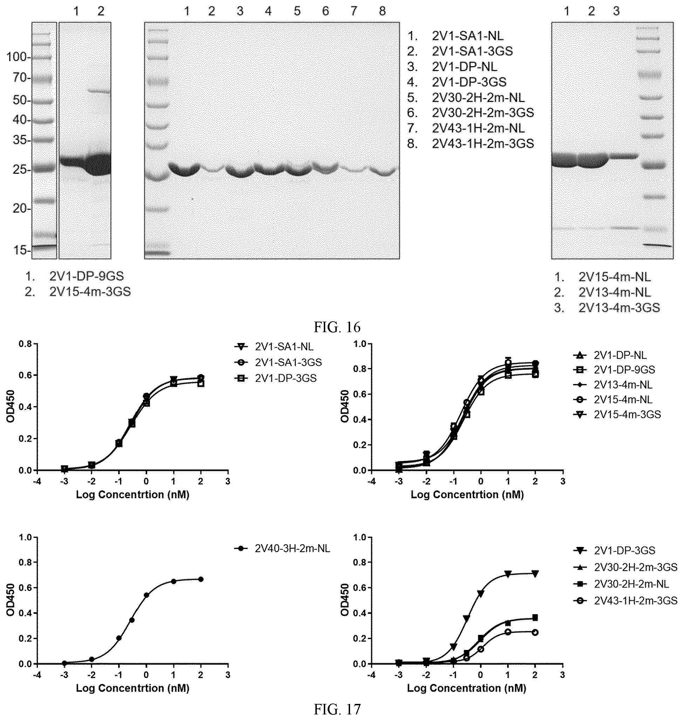

For the above bivalent nanobodies, the plasmid construction, expression and purification methods were the same as those described in "1. Bivalentization design of nanobodies" in Example 1. The Coomassie-stained gel images of some of the proteins after purification are shown in FIG. 16. The molecular weight of the target protein was about 30 kDa.

**2. Assay for bivalentization activity of humanized nanobodies**

The purified bivalent humanized nanobodies were evaluated for their VEGFA binding activity, and the experimental procedure was the same as that described in "2. Assay for VEGFA binding activity of bivalent nanobodies" in Example 1. The ELISA results for the bivalent humanized nanobodies binding to VEGFA are shown in FIG. 17. The EC50 results of the ELISA are shown in Table 17. The results show that most of the bivalent humanized nanobodies can efficiently bind to VEGFA.

Table 17. EC50 results of ELISA for bivalent humanized nanobodies binding to VEGFA

| Bivalent nanobody | EC50 (nM) for binding to VEGFA |
|---|---|
| 2V1-SA1-NL | 0.28 |
| 2V1-SA1-3GS | 0.26 |
| 2V1-DP-NL | 0.20 |
| 2V1-DP-3GS | 0.27 |
| 2V1-DP-9GS | 0.23 |
| 2V13-4m-NL | 0.22 |
| 2V15-4m-NL | 0.19 |
| 2V15-4m-3GS | 0.24 |
| 2V30-2H-2m-NL | 0.81 |
| 2V30-2H-2m-3GS | 0.94 |
| 2V40-3H-2m-NL | 0.26 |

(continued)

| Bivalent nanobody | EC50 (nM) for binding to VEGFA |
|---|---|
| 2V43-1H-2m-3GS | 1.19 |

**Example 7: Preparation and Activity Study of Monovalent Nanobody-Fc Fusion Proteins**

**1. Preparation of monovalent nanobody-Fc fusion proteins**

To achieve the bivalentization of nanobodies, in addition to linearly and tandemly linking 2 nanobodies, dimerization domains or fragments, such as the Fc of IgG1 antibody (SEQ ID NO: 134), PPCP-Fc (SEQ ID NO: 158, i.e., the remaining amino acid sequence after removal of DKTHTC in SEQ ID NO: 134), or IgG1 Fc mutant Fc-m1 (SEQ ID NO: 135), can also be utilized to link 2 nanobodies within the same molecule.

The amino acid sequence of the IgG1 Fc is as follows:

DKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKLTVDKSRWQQGNVFS
CSVMHEALHNHYTQKSLSLSPGK(SEQ ID NO:134)

The amino acid sequence of the PPCP-Fc is as follows:

PPCPAPELLGGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPSRDELTKN
QVSLTCLVKGFYPSDIAVEWESNGQPENNYKTTPPVLDSDGSFFLYSKLTVDKSRWQQGNVFSCSVM
HEALHNHYTQKSLSLSPGK(SEQ ID NO:158)

The amino acid sequence of the IgG1 Fc mutant Fc-m1 is as follows:

DKTHTCPPCPAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPSRE
EMTKNQVSLTCLVKGFYPSDIAVEWESNGQPENNYKTTPPVLDSDGSFFLYSKLTVDKSRWQQGNVF
SCSVMHEALHNHYTQKSLSLSPGK(SEQ ID NO:135)

Therefore, the expression of the monovalent nanobody-Fc fusion proteins was carried out. The names and numbers of the amino acid sequences of the constructed nanobody-Fc fusion proteins are shown in Table 18.

Table 18. Names and numbers of amino acid sequences of nanobody-Fc fusion proteins

| SEQ ID NO:x | Monovalent nanobody-Fc fusion protein |
|---|---|
| 136 | V15-PPCP-Fc |
| 137 | V15-9GS-PPCP-Fc |
| 138 | V15-Fc |
| 139 | V1-SA1-Fc-m1 |

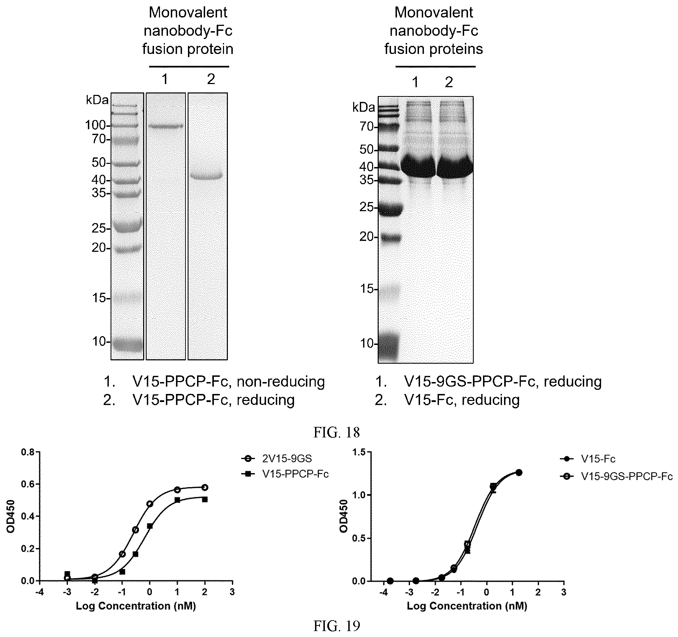

To facilitate the comparison of the VEGFA binding activity between the linear tandem bivalent nanobody 2V15-9GS (which contains a 6-histidine tag) and the fusion proteins V15-PPCP-Fc, V15-9GS-PPCP-Fc, and V15-Fc, a linker (SGGS (SEQ ID NO: 153)) and a 6-histidine tag (HHHHHH (SEQ ID NO: 154)) were added to the C-terminus of the Fc segment in the fusion proteins. During the plasmid construction for the monovalent nanobody-Fc fusion proteins, the nanobody and the Fc containing the linker and histidine tag were amplified separately using the PCR method, with the PCR template for the Fc fragment derived from a genetically synthesized Fc gene. A two-fragment homologous recombination method was used to integrate the nanobody and Fc into the vector pCDNA3.1(+), and the enzyme cutting sites used were NheI and XbaI. The signal peptide of the fusion protein was METDTLLLWVLLLWVPGSTG (SEQ ID NO: 155), followed by

**EP 4 574 846 A1**

the fusion of the target protein sequence. After successful sequencing of these fusion protein plasmids, endotoxin-free plasmid extraction was performed. Polyethylenimine (abbreviated as PEI, linear, MW = 25 kDa, purchased from Polysciences, Cat. No.: 23966-1), a transfection reagent, was used to perform transient transfection on 293F suspension cells. The cell supernatant was collected on day 4 or day 5 after transfection, and Ni-bead purification was carried out. The purified proteins were analyzed by SDS-PAGE, and under reducing conditions, the molecular weight of the protein monomers was about 39 kDa, which was in line with the expected value. The Coomassie-stained gel images of some of the monovalent nanobody-Fc fusion proteins after purification are shown in FIG. 18.

For the fusion protein (V1-SA1-Fc-m1) of the humanized antibody V1-SA1 with Fc, the amino acid sequence of the linker between V1-SA1 and Fc-m1 is as follows: EPKSA (SEQ ID NO: 156). An expression plasmid was constructed using the method described above, but without additional amino acids at the C-terminus of Fc, meaning that the fusion protein V1-SA1-Fc-m1 contained a Fc tag but no histidine tag. After transient transfection of 293F cells with the V1-SA1-Fc-m1 expression plasmid, Protein A bead affinity purification was performed. During protein elution, 0.1 M citric acid (pH 3.0) was used to elute the target protein, followed by rapid neutralization to obtain the target protein.

## 2. Activity assay for monovalent nanobody-Fc fusion proteins

The purified monovalent nanobody-Fc fusion proteins were subjected to VEGFA binding activity assay, and the ELISA experimental procedure was the same as that described in "2. Assay for VEGFA binding activity of bivalent nanobodies" in Example 1. The ELISA results are shown in FIG. 19, and the EC50 values are shown in Table 19.

Table 19. Assay for VEGFA binding activity of monovalent nanobody-Fc fusion proteins or tandem bivalent nanobodies

| Monovalent nanobody-Fc fusion protein or tandem bivalent nanobody | EC50 (nM) for binding to VEGFA |
| --- | --- |
| V15-PPCP-Fc | 0.64 |
| V15-9GS-PPCP-Fc | 0.35 |
| V15-Fc | 0.43 |
| 2V15-9GS | 0.25 |

As can be seen from the data in the table, the tested monovalent nanobody-Fc fusion proteins and linear tandem bivalent nanobodies both had strong VEGFA binding activity.

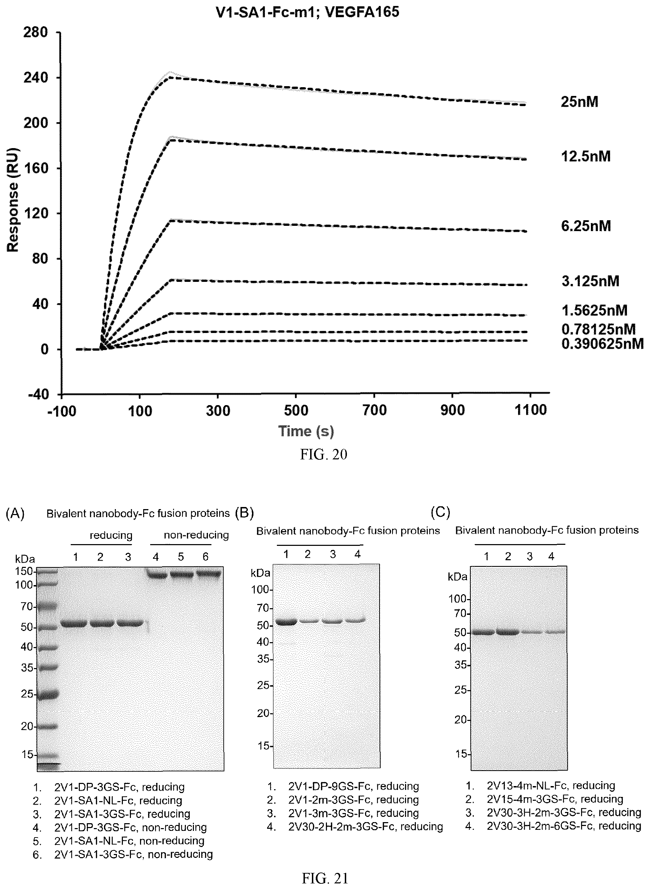

The surface plasmon resonance (SPR) technology was utilized to detect the affinity of the monovalent nanobody-Fc fusion protein (V1-SA1-Fc-m1) for VEGFA. Using the Biacore 8K instrument (Cytiva), V1-SA1-Fc-m1 as the ligand was captured onto a Protein A chip. During immobilization, V1-SA1-Fc-m1 was diluted to a protein concentration of 2 μg/mL with the mobile phase buffer HBS-EP (0.01 M HEPES pH 7.4, 0.15 M NaCl, 3 mM EDTA, 0.005% v/v Tween 20), and its coupling level was about 590 RU. During the experiment, the analyte was the antigen VEGFA165 (label-free, purchased from GenScript, Cat. No.: Z03073) with the dilution concentrations of 25 nM, 12.5 nM, 6.25 nM, 3.125 nM, 1.5625 nM, 0.78125 nM, and 0.390625 nM. In the kinetic analysis, the binding time between the fusion protein and the antigen VEGFA165 was 180 s, the dissociation time was 900 s, and the flow rate was 30 μL/min. After each "binding-dissociation" cycle, the chip was regenerated under the condition of 10 mM glycine hydrochloride (pH 1.5). The binding and dissociation curves of the fusion protein V1-SA1-Fc-m1 with the antigen VEGFA165 (FIG. 20) were fitted using the "1:1 binding" model to obtain the binding rate (ka), dissociation rate (kd), and equilibrium dissociation constant (KD), as shown in Table 20.

Table 20. Binding rate (ka), dissociation rate (kd), and equilibrium dissociation constant (KD) of fusion protein V1-SA1-Fc-m1 with antigen VEGFA165

| Ligand | Analyte | ka (1/Ms) | kd (1/s) | KD (M) |
| --- | --- | --- | --- | --- |
| V1-SA1-Fc-m1 | VEGFA165 | 8.77E+05 | 1.30E-04 | 1.48E-10 |

It can be seen from the SPR data that the monovalent nanobody-Fc fusion protein V1-SA1-Fc-m1 had a high affinity for VEGFA165 (KD = 0.148 nM).

## Example 8: Preparation and Activity Study of Bivalent Nanobody-Fc Fusion Proteins

**1. Expression and purification of bivalent nanobody-Fc fusion proteins**

In Example 7, the monovalent nanobody-Fc fusion protein showed a high affinity for VEGFA, and the results of Example 2 indicate that the VEGFA binding activity of bivalent nanobodies was higher than that of monovalent nanobodies. Therefore, bivalent nanobody-Fc fusion proteins were constructed by fusing the bivalent nanobodies from Table 16 with IgG1 Fc (SEQ ID NO: 134) for expression. The names and numbers of the amino acid sequences of some of the bivalent nanobody-Fc fusion proteins are shown in Table 21.

Table 21. Names and numbers of amino acid sequences of bivalent nanobody-Fc fusion proteins

| SEQ ID NO:x | Name of bivalent nanobody-Fc fusion protein |
|---|---|
| 140 | 2V1-SA1-NL-Fc |
| 141 | 2V1-SA1-3GS-Fc |
| 142 | 2V1-DP-3GS-Fc |
| 143 | 2V1-DP-9GS-Fc |
| 144 | 2V1-2m-3GS-Fc |
| 145 | 2V1-3m-3GS-Fc |
| 146 | 2V13-4m-NL-Fc |
| 147 | 2V15-4m-3GS-Fc |
| 148 | 2V30-2H-2m-3GS-Fc |
| 149 | 2V30-3H-2m-3GS-Fc |
| 150 | 2V30-3H-2m-6GS-Fc |

Expression plasmids for the above bivalent nanobody-Fc fusion proteins were constructed, followed by transient transfection and purification.

Specific procedure: Based on the amino acid sequences of the fusion proteins listed in the table, expression plasmids were constructed using gene synthesis or PCR method. For the PCR method, 2 identical nanobody genes and the Fc were integrated into the vector pCDNA3.1 (+), and the enzyme cutting sites used were NheI and XbaI. The signal peptide of the fusion protein was: METDTLLLWVLLLWVPGSTG (SEQ ID NO: 155), followed by the fusion of the target protein sequence. After sequencing confirmation and endotoxin-free plasmid extraction, polyethylenimine (abbreviated as PEI, linear, MW = 25 kDa, purchased from Polysciences, Cat. No.: 23966-1), a transfection reagent, was used to perform transient transfection on 293F suspension cells. The cell supernatant was collected on day 4 or day 5 after transfection, and Protein A bead affinity purification was carried out. During protein elution, 0.1 M citric acid (pH 3.0) was used to elute the target protein. The protein elution product was added dropwise to 0.5 volumes of 1 M sodium citrate (pH = 8.5) for rapid neutralization. After concentration and buffer exchange, the protein was used for subsequent testing.

The Coomassie-stained gel images of the exemplary bivalent nanobody-Fc fusion proteins after purification are shown in FIG. 21. Under reducing SDS-PAGE conditions, the protein molecular weight was about 53 kDa.

**2. Activity study of bivalent nanobody-Fc fusion proteins**

The VEGFA binding activity of the purified bivalent nanobody-Fc fusion proteins was detected by ELISA. The ELISA was conducted as follows: Human VEGFA165 (label-free, purchased from Sino Biological, Cat. No.: HPLC-10008-HNAH) was diluted with ELISA coating buffer to a final concentration of 0.3 $\mu$g/mL and added to a microplate for coating at 4 °C overnight. After the plate was blocked with 5% skim milk powder (prepared with PBST), different concentrations of fusion protein (maximum concentration of 100 nM, minimum concentration of 0.0001 nM, 10-fold serial dilutions, prepared with PBST) were added, and the plate was incubated at 37 °C for 1 h and washed 3 times with PBST. Next, a detection antibody recognizing the Fc tag, HRP-labeled Goat Anti-Human IgG antibody (purchased from Abbkine, Cat. No.: A21050), was added, and the plate was further incubated at room temperature for 45 min and washed again. Subsequently, 100 $\mu$L of TMB (purchased from Tiangen Biotech, Cat. No.: PA107-01) was added to each well for color development at 37 °C for 15 min, followed by the addition of 50 $\mu$L of stopping solution. The absorbance at 450 nm (OD450) was measured using a microplate reader.

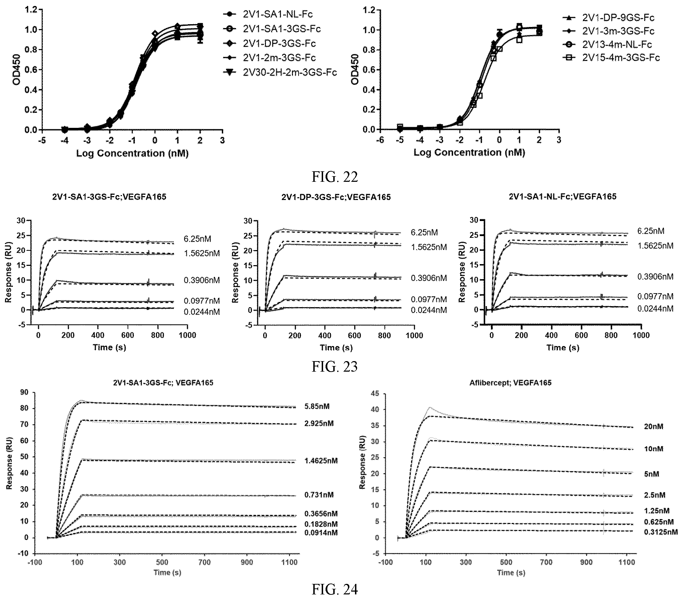

The ELISA results for some of the fusion proteins are shown in FIG. 22, where Negative control represents an IgG1 Fc fusion protein that binds to an irrelevant target. The EC50 values are shown in Table 22.

Table 22. EC50 values of fusion proteins for binding to VEGFA

| Name of bivalent nanobody-Fc fusion protein | EC50 (nM) for binding to VEGFA |
|---|---|
| 2V1-SA1-NL-Fc | 0.16 |
| 2V1-SA1-3GS-Fc | 0.13 |
| 2V1-DP-3GS-Fc | 0.14 |
| 2V1-DP-9GS-Fc | 0.11 |
| 2V1-2m-3GS-Fc | 0.14 |
| 2V1-3m-3GS-Fc | 0.11 |
| 2V13-4m-NL-Fc | 0.13 |
| 2V15-4m-3GS-Fc | 0.17 |
| 2V30-2H-2m-3GS-Fc | 0.15 |
| Negative control | / |

The surface plasmon resonance (SPR) technology was utilized to determine the affinity of the bivalent nanobody-Fc fusion proteins (2V1-SA1-3GS-Fc, 2V1-SA1-NL-Fc, and 2V1-DP-3GS-Fc) for VEGFA165. Using the Biacore 8K instrument (Cytiva) in the "low immobilization" mode, VEGFA165 (label-free, purchased from Sino Biological, Cat. No.: HPLC-10008-HNAH) was coupled onto a CM5 chip (due to the high affinity and slow dissociation of these proteins with VEGFA165, the coupling level of VEGFA165 was reduced). Each fusion protein was diluted with the mobile phase buffer HBS-EP (0.01 M HEPES pH 7.4, 0.15 M NaCl, 3 mM EDTA, 0.005% v/v Tween 20) to the dilution concentrations of 0.0244 nM, 0.0977 nM, 0.3906 nM, 1.5625 nM, and 6.25 nM. In the kinetic analysis, the binding time between the antigen and the antibody was 120 s, the dissociation time was 780 s, and the flow rate was 40 $\mu$L/min. In a multi-cycle mode, the chip was regenerated with 100 mM hydrochloric acid. The binding and dissociation curves of the fusion proteins with VEGFA165 were fitted using the "1:1 binding" model to obtain the binding rate (ka), dissociation rate (kd), and equilibrium dissociation constant (KD).

The binding and dissociation curves of the bivalent nanobody-Fc fusion proteins with VEGFA165 are shown in FIG. 23, and the fitted KD is shown in Table 23.

Table 23. Binding rate (ka), dissociation rate (kd), and equilibrium dissociation constant (KD) of bivalent nanobody-Fc fusion proteins with VEGFA165

| Ligand | Name of analyte protein | ka (1/Ms) | kd (1/s) | KD (M) |
|---|---|---|---|---|
| VEGFA165 | 2V1-SA1-3GS-Fc | 1.01E+07 | 6.88E-05 | 6.83E-12 |
| VEGFA165 | 2V1-SA1-NL-Fc | 1.29E+07 | 4.55E-05 | 3.53E-12 |
| VEGFA165 | 2V1-DP-3GS-Fc | 1.14E+07 | 4.38E-05 | 3.84E-12 |

It can be seen from Table 23 that the 3 bivalent nanobody-Fc fusion proteins had high affinity for VEGFA165 (KD = 3.84-6.83 pM).

In addition, the KD of the fusion proteins with VEGFA165 was retested using human VEGFA165 antigens from different reagent suppliers. Using a method similar to the SPR experiment described above, the KD values of the fusion protein (2V1-SA1-3GS-Fc) and the positive control drug aflibercept with VEGFA165 were determined. Specifically, using the Biacore 8K instrument (Cytiva) in the "low immobilization" mode, VEGFA165 (label-free, purchased from GenScript, Cat. No.: Z03073) was coupled onto a CM5 chip. The proteins were diluted with the mobile phase buffer HBS-EP. The dilution concentrations of 2V1-SA1-3GS-Fc were 0.091 nM, 0.1828 nM, 0.3656 nM, 0.73125 nM, 1.4625 nM, 2.925 nM, and 5.85 nM. The dilution concentrations of aflibercept were 0.3125 nM, 0.625 nM, 1.25 nM, 2.5 nM, 5 nM, 10 nM, and 20 nM. In the kinetic analysis, the binding time between the antigen and the antibody was 150 s, the dissociation time was 1000 s, and the flow rate was 30 $\mu$L/min. In a multi-cycle mode, the chip was regenerated with 10 mM glycine hydrochloride (pH = 1.5). The binding and dissociation curves of the proteins to be tested with VEGFA165 were fitted using the "1:1 binding" model to obtain the binding rate (ka), dissociation rate (kd), and equilibrium dissociation constant (KD), as shown in Table 24. The binding and dissociation curves (SPR) of the bivalent nanobody-Fc fusion protein (2V1-SA1-3GS-Fc) and aflibercept with VEGFA165 are shown in FIG. 24.

Table 24. Binding rate (ka), dissociation rate (kd), and equilibrium dissociation constant (KD) of 2V1-SA1-3GS-Fc and aflibercept with VEGFA165

| Ligand | Name of analyte protein | ka (1/Ms) | kd (1/s) | KD (M) |
|---|---|---|---|---|
| VEGFA165 | 2V1-SA1-3GS-Fc | 8.44E+06 | 3.95E-05 | 4.68E-12 |
| VEGFA165 | Aflibercept | 1.64E+06 | 9.57E-05 | 5.84E-11 |

By comparing the KD values in Table 23 and Table 24, it can be seen that the fusion protein 2V1-SA1-3GS-Fc had extremely high affinity for human VEGFA165 from different reagent suppliers, with KD values at the pM level; the KD values were significantly lower than those of aflibercept (about 1/12 of aflibercept's KD), indicating that the fusion protein 2V1-SA1-3GS-Fc had a higher affinity for VEGFA165 compared to aflibercept.

SPR tests were conducted using a similar method as described above (coupling the antigen VEGFA165) to compare the KD values of the monovalent nanobodies and bivalent nanobodies with VEGFA (Table 7) and the KD values of the above bivalent nanobody-Fc fusion proteins with VEGFA (Table 23 and Table 24). The KD values of the antibodies in different construction formats with VEGFA were summarized as shown in Table 25.

Table 25. Summarized KD values of antibodies in different construction formats with VEGFA

| Nanobody construction format | Average KD value (pM) | Fold increase in antibody-antigen affinity |
|---|---|---|
| Monovalent nanobody | 496 | 1 |
| Bivalent nanobody | 22 | 22.5 |
| Bivalent nanobody-Fc fusion protein | 4.7 | 105.5 |

From Table 25, it can be seen that compared to the monovalent nanobodies, the bivalent nanobodies and the bivalent nanobody-Fc fusion proteins exhibited 22.5-fold and 105.5-fold increase in affinity for the antigen, respectively. Therefore, as the number of antibody or antigen-binding fragments in a protein molecule increases, the affinity of the protein molecule for the antigen will increase significantly.

The cross-binding activity of 2V1-SA1-3GS-Fc with VEGFA proteins from different species was detected using the ELISA method. Mouse VEGFA164 was purchased from Sino Biological (Cat. No.: 50159-MNAB), and rat VEGFA164 was purchased from Sino Biological (Cat. No.: 80006-RNAB). Rabbit VEGFA121 was expressed in transiently transfected 293F cells and purified using Ni beads. The nucleotide sequence of rabbit VEGFA121 was synthesized and cloned into the vector pCDNA3.1(+). The amino acid sequence of rabbit VEGFA121 is: APMAEEGDNKPHEVVKFMEVYRRSYCQPI ETLVDIFQEYPDEIEYIFKPSCVPLVRCGGCCNDESLECV PTEEFNVTMQIMRIKPHQGQHIGEMSFLQHNKCECRPK KDRARQEKCDKPRR (SEQ ID NO: 157). The protein's N-terminus adopts its own signal peptide, while its C-terminus is sequentially fused with a flexible linker (GGGGS (SEQ ID NO: 152)) and a 6-histidine tag (HHHHHH (SEQ ID NO: 154)).

The ELISA experimental procedure used was similar to that described in "2. Assay for VEGFA binding activity of bivalent nanobodies" in Example 1. Different concentrations of 2V1-SA1-3GS-Fc were added to ELISA plates coated with different antigens, and the plates were incubated for 1 h. A detection antibody recognizing the Fc tag, HRP-labeled Goat Anti-Human IgG antibody (purchased from Abbkine, Cat. No.: A21050), was added, and the plates were further incubated at room temperature for 45 min, followed by washing, TMB color development, and reaction termination. The OD450 readings were taken using a microplate reader.

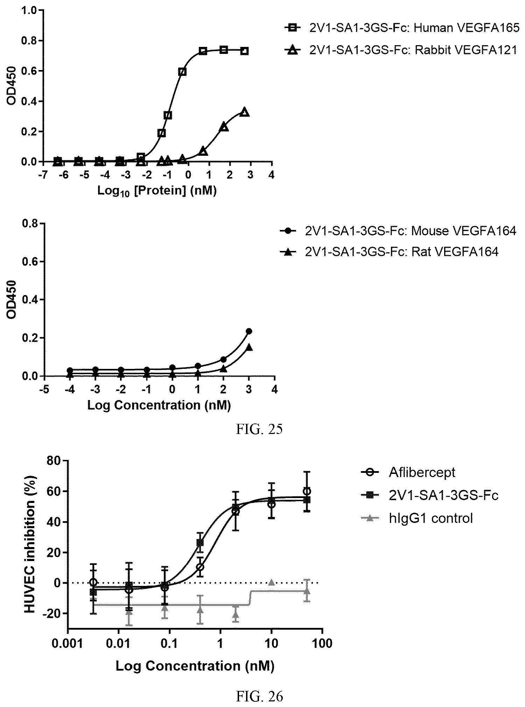

The ELISA data for binding to VEGFA are shown in FIG. 25, and the EC50 values are shown in Table 26.

Table 26. EC50 or maximum binding values of 2V1-SA1-3GS-Fc for binding to VEGFA from different species

| VEGFA from different species | EC50 (nM) for binding to VEGFA | Maximum binding value |
|---|---|---|
| Human VEGFA165 | 0.14 | 0.74 |
| Rabbit VEGFA121 | 23.72 | 0.51 |
| Mouse VEGFA164 | / | / |
| Rat VEGFA164 | / | / |

From the above, it can be seen that 2V1-SA1-3GS-Fc has relatively high binding activity only to human VEGFA165, can bind weakly to rabbit VEGFA121, and cannot bind to mouse and rat VEGFA164.

Using a primary cell HUVEC proliferation inhibition experiment, the efficacy of the fusion protein 2V1-SA1-3GS-Fc at the cellular level, i.e., its activity in blocking the VEGFA signaling pathway, was validated. In the experiment, the positive control was the marketed drug aflibercept, and the negative control was a human IgG1 antibody (hIgG1 control) that binds to HIV. The number of viable HUVECs was determined using the CellTiter-Glo luminescent cell viability assay. Experimental procedure: HUVECs (purchased from ScienCell, Cat. No.: 8000) were routinely passaged using a complete medium containing ECM + 5% FBS + 1% ECGS (purchased from ScienCell, Cat. Nos.: 1001, 0010, and 5352 for the 3 reagents, respectively). At the beginning of the experiment, the protein to be tested was serially diluted 5-fold with a low-serum medium (ECM + 1% FBS + 0.05% ECGS) to the desired concentrations ranging from 1000 nM to 0.0128 nM (the final antibody concentrations during HUVEC incubation were 250 nM to 0.0032 nM). Each antibody dilution was mixed with an equal volume of a 200 ng/mL VEGFA165 solution, and the mixture was incubated in a cell incubator at 37 °C for 2 h. The mixture was added to a 96-well plate (50 $\mu$L/well). Subsequently, the HUVECs were resuspended in a low-serum medium and transferred to the 96-well plate at a volume of 50 $\mu$L/well (ensuring $6 \times 10^3$ HUVECs per well). The plate wells containing cells, VEGF, and protein dilution were labeled as Cell+VEGF+Protein. Meanwhile, control groups were set: one with only cells but without VEGF and protein (Cell only, expected to have the slowest cell proliferation), and another with cells and VEGF but without protein (Cell and VEGF only, expected to have the fastest cell proliferation). The 96-well plates were incubated in an incubator at 37 °C with 5% $CO_2$ for 3 days. After the incubation was completed, the CellTiter-Glo reagent (purchased from Promega, Cat. No.: G7573) was added to each well, and the mixture was mixed well. The luminescence intensity was measured by a microplate reader (Envision model, PerkinElmer). The IC50 values were calculated using GraphPad Prism 9 software through four-parameter nonlinear regression analysis.

The experimental results are shown in FIG. 26. The fitted IC50 values and maximum inhibition rates are shown in Table 27. The formula for calculating the inhibition rate is as follows: Inhibition (%) = {1 - [(Cell+VEGF+Protein) - (Cell only) / (Cell and VEGF only) - (Cell only)]} $\times$ 100

Table 27. IC50 values and maximum inhibition rates for HUVEC proliferation inhibition

| Protein | HUVEC proliferation inhibition | |
| --- | --- | --- |
| | IC50 (nM) | Maximum inhibition rate (%) |
| Aflibercept | 0.81 | 60 |
| 2V1-SA1-3GS-Fc | 0.38 | 55 |
| hIgG1 control | NA | 0 |

Therefore, it can be seen that the IC50 of the fusion protein 2V1-SA1-3 GS-Fc for inhibiting HUVEC proliferation was about half of that of the positive control aflibercept. This indicates that the fusion protein 2V1-SA1-3GS-Fc could efficiently block the downstream signaling pathway activated by VEGFA at the cellular level and thus inhibit VEGFA-stimulated HUVEC proliferation, and the activity of the fusion protein 2V1-SA1-3GS-Fc at the cellular level was stronger than that of the positive control aflibercept.

The preferred embodiments of the present disclosure are described in detail above, which, however, are not intended to limit the present disclosure. Within the scope of the technical conception of the present disclosure, various simple modifications can be made to the technical solutions of the present disclosure, all of which will fall within the protection scope of the present disclosure.

In addition, it should be noted that the various specific technical features described in the above specific embodiments can be combined in any suitable manner in which the features do not contradict each other. In order to avoid unnecessary repetition, such combinations will not be illustrated separately.

**Claims**

1. An anti-VEGFA fusion protein, comprising two or more anti-VEGFA antibodies or antigen-binding fragments thereof, and a Fc fragment, wherein the anti-VEGFA antibody or the antigen-binding fragment thereof comprising CDR-H1, CDR-H2, and CDR-H3 of a heavy chain variable region, wherein

an amino acid sequence of the CDR-H1 comprises SYTMG (SEQ ID NO: 1) or an amino acid sequence having at least 80% identity to SYTMG (SEQ ID NO: 1);
an amino acid sequence of the CDR-H2 comprises AISKGGYKYX$_1$X$_2$VSLEA (SEQ ID NO: 2) or an amino acid sequence having at least 80% identity to AISKGGYKYX$_1$X$_2$VSLEA (SEQ ID NO: 2);
an amino acid sequence of the CDR-H3 comprises TRAYGSSRLX$_3$LAX$_4$TYEY (SEQ ID NO: 3) or an amino acid sequence having at least 80% identity to TRAYGSSRLX$_3$LAX$_4$TYEY (SEQ ID NO: 3).

2. The anti-VEGFA fusion protein according to claim 1, wherein the anti-VEGFA antibody or the antigen-binding fragment thereof is directly or indirectly connected to another anti-VEGFA antibody or antigen-binding fragment thereof, and/or the Fc fragment.

3. The anti-VEGFA fusion protein according to claim 2, wherein the N-terminus, C-terminus, and/or internal residues of the anti-VEGFA antibody or the antigen-binding fragment thereof are connected to the N-terminus, C-terminus, and/or internal residues of another anti-VEGFA antibody or antigen-binding fragment thereof or the Fc fragment.

4. The anti-VEGFA fusion protein according to any one of claims 1-3, wherein the two or more anti-VEGFA antibodies or antigen-binding fragments thereof are anti-VEGFA antibodies or antigen-binding fragments thereof having identical sequences, partially identical sequences, or completely different sequences.

5. The anti-VEGFA fusion protein according to any one of claims 1-4, wherein $X_1X_2$ in SEQ ID NO: 2 represents DS, DA, NT, DT, NA, or NS; $X_3$ in SEQ ID NO: 3 represents R or K, and $X_4$ represents D, N, E, or K.

6. The anti-VEGFA fusion protein according to any one of claims 1-5, wherein the amino acid sequences of the CDR-H1, the CDR-H2, and the CDR-H3 comprise any one of the following groups:

   A) SEQ ID NOs: 1, 4, and 9;
   B) SEQ ID NOs: 1, 5, and 9;
   C) SEQ ID NOs: 1, 6, and 10;
   D) SEQ ID NOs: 1, 4, and 11;
   E) SEQ ID NOs: 1, 7, and 12;
   F) SEQ ID NOs: 1, 6, and 11;
   G) SEQ ID NOs: 1, 4, and 10;
   H) SEQ ID NOs: 1, 5, and 12;
   I) SEQ ID NOs: 1, 4, and 12;
   J) SEQ ID NOs: 1, 8, and 12; and
   K) SEQ ID NOs: 1, 33, and 34.

7. The anti-VEGFA fusion protein according to any one of claims 1-6, wherein the anti-VEGFA antibody or the antigen-binding fragment thereof is a nanobody, a chimeric antibody, a Fab fragment, a Fab' fragment, a Fd fragment, a Fd' fragment, a Fv fragment, a dAb fragment, an isolated CDR, a F(ab')$_2$ fragment, a single domain antibody, a single chain antibody molecule, or a linear antibody.

8. The anti-VEGFA fusion protein according to any one of claims 1-7, wherein an amino acid sequence of the anti-VEGFA antibody or the antigen-binding fragment thereof comprises any one of the amino acid sequences of SEQ ID NOs: 13-32 and 35-61, or has at least 80% identity to any one of the amino acid sequences of SEQ ID NOs: 13-32 and 35-61.

9. The anti-VEGFA fusion protein according to any one of claims 1-8, wherein an amino acid sequence of the anti-VEGFA fusion protein comprises any one of the amino acid sequences of SEQ ID NOs: 62-76, 92-133, 136-150, and 159, or has at least 80% identity to any one of the amino acid sequences of SEQ ID NOs: 62-76, 92-133, 136-150, and 159.

10. A chimeric antigen receptor, wherein an extracellular domain of the chimeric antigen receptor comprises the anti-VEGFA fusion protein according to any one of claims 1-9.

11. A nucleic acid encoding the anti-VEGFA fusion protein according to any one of claims 1-9 or the chimeric antigen receptor according to claim 10.

12. A vector, comprising the nucleic acid according to claim 11.

13. A cell, comprising the nucleic acid according to claim 11 or the vector according to claim 12.

14. An immune cell expressing the anti-VEGFA fusion protein according to any one of claims 1-9 or the chimeric antigen receptor according to claim 10.

15. A preparation method for an anti-VEGFA fusion protein, comprising introducing the nucleic acid according to claim 11 into a host cell, and inducing expression thereof.

16. A product for treating and/or diagnosing a disease, comprising any one of the following:

   A) the anti-VEGFA fusion protein according to any one of claims 1-9;
   B) the chimeric antigen receptor according to claim 10;
   C) the nucleic acid according to claim 11;
   D) the vector according to claim 12;
   E) the cell according to claim 13; or
   F) the immune cell according to claim 14.

17. Use of the anti-VEGFA fusion protein according to any one of claims 1-9, the chimeric antigen receptor according to claim 10, the nucleic acid according to claim 11, the vector according to claim 12, the cell according to claim 13, the immune cell according to claim 14, or the product for treating and/or diagnosing a disease according to claim 16 in blocking the VEGFA signaling pathway, wherein
preferably, the use comprises:

   A) use in preparing a medicament for treating and/or preventing a tumor;
   B) use in preparing a medicament for treating and/or preventing an ophthalmic disease involving angiogenesis (e.g., a fundus vascular disease), wherein the fundus vascular disease is preferably age-related macular degeneration (AMD), diabetic macular edema (DME), diabetic retinopathy, central retinal vein occlusion, pathological myopia, neovascular glaucoma, etc.; and/or
   C) use in inhibiting abnormal vascular proliferation, preferably blocking vascular endothelial cell proliferation.

18. A method for treating and/or preventing a disease, comprising administering to an individual the anti-VEGFA fusion protein according to any one of claims 1-9, the chimeric antigen receptor according to claim 10, the nucleic acid according to claim 11, the vector according to claim 12, the cell according to claim 13, the immune cell according to claim 14, or the product for treating and/or diagnosing a disease according to claim 16.

19. A method for treating and/or preventing a disease, comprising contacting the anti-VEGFA fusion protein according to any one of claims 1-9, the chimeric antigen receptor according to claim 10, the nucleic acid according to claim 11, the vector according to claim 12, the cell according to claim 13, the immune cell according to claim 14, or the product for treating and/or diagnosing a disease according to claim 16 with a target cell.

20. The method according to claim 19, wherein the target cell is selected from cells expressing VEGFA, such as cardiomyocytes, renal proximal tubule cells, hepatocytes, vascular endothelial cells, granulosa cells, specialized epithelial cells, mesenchymal cells, macrophages, platelets, dendritic cells, activated T cells, retinal pigment epithelial cells, Muller cells in the retina, astrocytes, osteoblasts, bronchial and alveolar epithelial cells, pericytes, vascular smooth muscle cells, myofibroblasts, keratinocytes, renal mesangial cells, tumor cells, etc.

21. The method according to any one of claims 18-20, wherein the disease is a disease associated with the VEGFA signaling pathway; preferably, the disease can be a tumor, abnormal vascular proliferation, or an ophthalmic disease involving angiogenesis.

22. A method for blocking VEGFA-mediated vascular endothelial cell proliferation or inhibiting angiogenesis, comprising contacting a vascular endothelial cell with the anti-VEGFA fusion protein according to any one of claims 1-9, the chimeric antigen receptor according to claim 10, the nucleic acid according to claim 11, the vector according to claim 12, the cell according to claim 13, the immune cell according to claim 14, or the product for treating and/or diagnosing a disease according to claim 16.

FIG. 1

FIG. 2

FIG. 3

FIG. 4

FIG. 5

Heterologous bivalent
nanobodies

1. V15-V40
2. V1-V15
3. V1-V30
4. V1-V40
5. V30-V40
6. V15-V30

FIG. 6

FIG. 7

FIG. 8

Bivalent nanobodies (2V1)

1. 2V1-NL
2. 2V1-3A
3. 2V1-6A

Bivalent nanobodies (2V15)

1. 2V15-9S
2. 2V15-20GS
3. 2V15-30GS
4. 2V15-39GS

Bivalent nanobodies (2V1 and 2V15)

1. 2V1-EAAAK-Turn
2. 2V15-EAAAK-Turn

FIG. 9

FIG. 10

FIG. 11

Left to stand at 4 °C for 30 days

1  2  3

1. 2V1-3GS
2. 2V1-(WL)2
3. 2V1-EAAAK-Turn

FIG. 12

FIG. 13

Left to stand at 40 °C for 7 days

1. 2V1-9GS
2. 2V1-30GS
3. 2V1-39GS
4. 2V1-(WL)2
5. 2V1-(AP)5
6. 2V1-(AP)10
7. 2V1-EAAAK-Rigid

FIG. 14

FIG. 15

1. 2V1-SA1-NL
2. 2V1-SA1-3GS
3. 2V1-DP-NL
4. 2V1-DP-3GS
5. 2V30-2H-2m-NL
6. 2V30-2H-2m-3GS
7. 2V43-1H-2m-NL
8. 2V43-1H-2m-3GS

1. 2V1-DP-9GS
2. 2V15-4m-3GS

1. 2V15-4m-NL
2. 2V13-4m-NL
3. 2V13-4m-3GS

FIG. 16

FIG. 17

Monovalent nanobody-Fc fusion protein

1. V15-PPCP-Fc, non-reducing
2. V15-PPCP-Fc, reducing

Monovalent nanobody-Fc fusion proteins

1. V15-9GS-PPCP-Fc, reducing
2. V15-Fc, reducing

FIG. 18

FIG. 19

FIG. 20

(A) Bivalent nanobody-Fc fusion proteins

1. 2V1-DP-3GS-Fc, reducing
2. 2V1-SA1-NL-Fc, reducing
3. 2V1-SA1-3GS-Fc, reducing
4. 2V1-DP-3GS-Fc, non-reducing
5. 2V1-SA1-NL-Fc, non-reducing
6. 2V1-SA1-3GS-Fc, non-reducing

(B) Bivalent nanobody-Fc fusion proteins

1. 2V1-DP-9GS-Fc, reducing
2. 2V1-2m-3GS-Fc, reducing
3. 2V1-3m-3GS-Fc, reducing
4. 2V30-2H-2m-3GS-Fc, reducing

(C) Bivalent nanobody-Fc fusion proteins

1. 2V13-4m-NL-Fc, reducing
2. 2V15-4m-3GS-Fc, reducing
3. 2V30-3H-2m-3GS-Fc, reducing
4. 2V30-3H-2m-6GS-Fc, reducing

FIG. 21

FIG. 22

FIG. 23

FIG. 24

FIG. 25

FIG. 26

## INTERNATIONAL SEARCH REPORT

| International application No. |
| --- |
| **PCT/CN2023/118594** |

| A. | CLASSIFICATION OF SUBJECT MATTER |
| --- | --- |

C07K19/00(2006.01)i; C12N15/62(2006.01)i; C12N5/10(2006.01)i; A61K39/395(2006.01)i

According to International Patent Classification (IPC) or to both national classification and IPC

| B. | FIELDS SEARCHED |
| --- | --- |

Minimum documentation searched (classification system followed by classification symbols)

IPC:C07K; C12N; A61K

Documentation searched other than minimum documentation to the extent that such documents are included in the fields searched

Electronic data base consulted during the international search (name of data base and, where practicable, search terms used)

CNTXT, USTXT, WOTXT, VEN, CNABS, PubMed, ISI Web of Knowledge, CNKI, NCBI, CJFD baidu, STN, 中国专利生物序列检索, China Patent Biological Sequence Search: VEGFA, 纳米抗体, 二价抗体, 抗原结合片段, Fc, 融合蛋白, CDR, 重链可变区, AMD, 二价纳米抗体-Fc融合蛋白, SEQ ID NOs: 1-76, 92-133, 136-150, 159, 血管异常增生, nanobody, bivalent antibody, antibody binding fragment, fusion protein, VH, bivalent nanobody-Fc fusion protein, vascular dysplasia

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | CN 103842380 A (GLAXO GROUP LTD.) 04 June 2014 (2014-06-04) abstract | 1-22 |
| A | CN 103965363 A (SHANGHAI ALLBRIGHT BIOTECHNOLOGY CO., LTD.) 06 August 2014 (2014-08-06) entire document | 1-22 |
| A | WO 2013014208 A2 (GLAXO GROUP LTD. et al.) 31 January 2013 (2013-01-31) entire document | 1-22 |
| A | CN 104592395 A (CHINA PHARMACEUTICAL UNIVERSITY) 06 May 2015 (2015-05-06) entire document | 1-22 |
| A | CN 112552410 A (SUNSHINE GUOJIAN PHARMACEUTICAL (SHANGHAI) CO., LTD.) 26 March 2021 (2021-03-26) entire document | 1-22 |

| ✓ Further documents are listed in the continuation of Box C. | ✓ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **04 December 2023** | **11 December 2023** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/118594** |

**C.    DOCUMENTS CONSIDERED TO BE RELEVANT**

| Category* | Citation of document, with indication, where appropriate, of the relevant passages | Relevant to claim No. |
| --- | --- | --- |
| A | KHODABAKHSH, F. et al. "Development of a Novel Nano-Sized Anti-VEGFA Nanobody with Enhanced Physicochemical and Pharmacokinetic Properties" *Artificial Cells, Nanomedicine, and Biotechnology,* Vol. 46, No. (7), 25 August 2017 (2017-08-25), pp. 1402-1414 | 1-22 |

Form PCT/ISA/210 (second sheet) (July 2022)

# EP 4 574 846 A1

## INTERNATIONAL SEARCH REPORT

International application No.

**PCT/CN2023/118594**

**Box No. I    Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT

| International application No. |
| --- |
| **PCT/CN2023/118594** |

**Box No. II      Observations where certain claims were found unsearchable (Continuation of item 2 of first sheet)**

This international search report has not been established in respect of certain claims under Article 17(2)(a) for the following reasons:

1. ☑ Claims Nos.: **17-22**
because they relate to subject matter not required to be searched by this Authority, namely:

Claims 17-22 relate to a use in blocking a VEGFA signaling pathway, a method for treatment and/or prevention of a disease, and a method for blocking VEGFA-mediated vascular endothelial cell proliferation or inhibiting angiogenesis, which fall within methods for treatment of diseases as defined in PCT Rule 39.1(iv). However, a search is still performed on the basis of the pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**EP 4 574 846 A1**

## INTERNATIONAL SEARCH REPORT
### Information on patent family members

International application No.

**PCT/CN2023/118594**

| Patent document cited in search report | | | Publication date (day/month/year) | Patent family member(s) | | | Publication date (day/month/year) |
|---|---|---|---|---|---|---|---|
| CN | 103842380 | A | 04 June 2014 | PE | 20141659 | A1 | 21 November 2014 |
| | | | | EP | 2736925 | A2 | 04 June 2014 |
| | | | | CA | 2842099 | A1 | 31 January 2013 |
| | | | | MA | 35352 | B1 | 01 August 2014 |
| | | | | US | 2017029494 | A1 | 02 February 2017 |
| | | | | WO | 2013014208 | A2 | 31 January 2013 |
| | | | | WO | 2013014208 | A3 | 10 May 2013 |
| | | | | DOP | 2014000017 | A | 30 April 2014 |
| | | | | EA | 201391812 | A1 | 30 June 2014 |
| | | | | EA | 028886 | B1 | 31 January 2018 |
| | | | | AU | 2012288846 | A1 | 06 February 2014 |
| | | | | AU | 2012288846 | B2 | 19 May 2016 |
| | | | | JP | 2014523746 | A | 18 September 2014 |
| | | | | JP | 5987057 | B2 | 06 September 2016 |
| | | | | US | 2014205604 | A1 | 24 July 2014 |
| | | | | US | 9499612 | B2 | 22 November 2016 |
| | | | | HK | 1198446 | A1 | 24 April 2015 |
| | | | | CO | 7020847 | A2 | 11 August 2014 |
| | | | | BR | 112014001855 | A2 | 21 February 2017 |
| | | | | CL | 2014000204 | A1 | 25 July 2014 |
| | | | | KR | 20140041908 | A | 04 April 2014 |
| | | | | KR | 101632620 | B1 | 23 June 2016 |
| | | | | MX | 2014001019 | A | 13 May 2014 |
| | | | | CR | 20140047 | A | 24 March 2014 |
| CN | 103965363 | A | 06 August 2014 | None | | | |
| WO | 2013014208 | A2 | 31 January 2013 | None | | | |
| CN | 104592395 | A | 06 May 2015 | None | | | |
| CN | 112552410 | A | 26 March 2021 | None | | | |

Form PCT/ISA/210 (patent family annex) (July 2022)

44

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9527925 B2